# EUROPEAN PATENT SPECIFICATION

(11) **EP 1 969 146 B1**
(45) Date of publication and mention of the grant of the patent: **06.10.2010**
(21) Application number: 07701933.9
(22) Date of filing: 04.01.2007
(51) Int. Cl.: C12N 15/10, C12N 15/64, C12N 15/66, C12Q 1/68

(54) **METHODS FOR NUCLEIC ACID MAPPING AND IDENTIFICATION OF FINE-STRUCTURAL-VARIATIONS IN NUCLEIC ACIDS AND UTILITIES**
VERFAHREN ZUR ZUORDNUNG VON NUKLEINSÄUREN UND ZUR IDENTIFIKATION FEIN STRUKTURIERTER VARIATIONEN IN NUKLEINSÄUREN SOWIE HILFSMITTEL DAFÜR
METHODES POUR LA CARTOGRAPHIE D'ACIDES NUCLEIQUES ET L'IDENTIFICATION DE VARIATIONS STRUCTURALES FINES DANS DES ACIDES NUCLEIQUES ET LEURS UTILISATIONS

(30) Priority: 04.01.2006 US 756417 P; 17.04.2006 US 792926 P; 15.06.2006 US 814378 P
(43) Date of publication of application: 17.09.2008
(73) Proprietor: Lok, Si, Hongkong (CN)
(72) Inventor: Lok, Si, Hongkong (CN)
(74) Representative: Meyer-Dulheuer, Karl-Hermann
(86) International application number: PCT/CN2007/000001
(87) International publication number: WO 2007/076726

(56) References cited:
- EP-A- 0 761 822
- EP-A- 1 533 386
- WO-A-01/79553
- WO-A-02/061097
- WO-A-03/044193
- WO-A-03/074734
- WO-A-2004/050918
- WO-A-2005/042781
- WO-A-2007/091077
- WO-A1-03/048395
- CN-A- 1 597 986
- CN-A- 1 678 753
- US-A- 5 753 462
- US-A1- 2003 186 251
- US-A1- 2004 241 852
- US-B1- 6 730 500
- SHENDURE JAY ET AL: "Accurate multiplex polony sequencing of an evolved bacterial genome" SCIENCE, AMERICAN ASSOCIATION FOR THE ADVANCEMENT OF SCIENCE, US, WASHINGTON, DC, vol. 309, no. 5741, 1 September 2005 (2005-09-01), pages 1728-1732, XP002427180 ISSN: 0036-8075
- TUZUN ERAY ET AL: "Fine-scale structural variation of the human genome" NATURE GENETICS, NATURE PUBLISHING GROUP, NEW YORK, US, vol. 37, no. 7, 1 July 2005 (2005-07-01), pages 727-732, XP002517280 ISSN: 1061-4036 [retrieved on 2005-05-15]
- SPINELLA D G ET AL: "Tandem arrayed ligation of expressed sequence tags (TALEST): a new method for generating global gene expression profiles" NUCLEIC ACIDS RESEARCH, OXFORD UNIVERSITY PRESS, SURREY, GB, vol. 27, no. 18, 15 September 1999 (1999-09-15), page e22, XP002304618 ISSN: 0305-1048
- WEI C-L ET AL: "5' Long serial analysis of gene expression (LongSAGE) and 3' LongSAGE for transcriptome characterization and genome annotation" PROCEEDINGS OF THE NATIONAL ACADEMY OF SCIENCES OF USA, NATIONAL ACADEMY OF SCIENCE, WASHINGTON, DC., US, vol. 101, no. 32, 10 August 2004 (2004-08-10), pages 11701-11706, XP002380745 ISSN: 0027-8424
- SAHA S ET AL: "USING THE TRANSCRIPTOME TO ANNOTATE THE GENOME" NATURE BIOTECHNOLOGY, NATURE PUBLISHING GROUP, NEW YORK, NY, US, vol. 20, no. 5, 1 May 2002 (2002-05-01), pages 508-512, XP001157218 ISSN: 1087-0156

## Description

### FIELD OF THE INVENTION

The present invention relates generally to methods for high-throughput analysis of fine structural variations in nucleic acids. In particular, the present invention relates to methods for nucleic acid mapping and identification of fine structural variations in nucleic acids and utilities, wherein two genomic variation tags sequence tags (GVTs) are juxtaposed and the two constituent members of the tag pair (GVT-pair) are unique positional markers of a defined separation distance in a target nucleic acid molecule, or the two constituent members of a tag pair are unique positional markers flanking two adjacent and cleavable restriction endonuclease sites.

### BACKGROUND OF THE INVENTION

While the most abundant type of variant in the human genome and the best-studied is the single-nucleotide polymorphism (SNP), it is increasingly clear that the so termed "fine-structural-variations" comprising alterations of copy number (insertions, deletions and duplications), inversions, translocations and other sequence rearrangements are integral features of the human and other nomes. These types of variations appear present in much greater frequency in the general population than originally thought. Evidence is mounting to indicate that structural variants can comprise millions of nucleotides of heterogeneity within every genome. Understanding the role of fine-structural-variations in genome evolution, interaction with the environment, phenotypic diversity and in disease or disease susceptibility are among the most actively investigated areas of current genomic research. For review, refer to Feuk et al (2006), Redon et al (2006), Check (2005), Cheng et al (2005), and Bailey et al (2002).

In comparison to analysis of SNPs, efficient high throughput methods for analysis of fine-structural-variations are not well developed. An important first step is the technique of array comparative genomic hybridization (array CGH) (Pinkel et al, 1998; Pinkel et al, U.S. Patent Nos. 5, 830, 645 and 6,159,685), which enables the qualification of relative copy numbers between target DNA and reference DNA. Array CGH allows reliable detection of deoxyribonucleic acid (DNA) copy-number differences between DNA or genomic samples with the resolution at the level of a single arrayed bacterial artificial chromosome (BAC) clone (Snijders et al, 2001; Albertson et al, 2000; Pinkel et al, 1998). The adaptation of array CGH to cDNA (Heiskanen et al, 2000; Pollack et al, 1999) and to high-density oligo-nucleotide array platforms (Bignell et al, 2004; Brennan et al, 2004; Huang et al, 2004; Lucito et al, 2003) further extends the resolution and utility of this approach. Through its use, array CGH has led to the identification of gene copy number alterations that are associated with tumor (Pinkel and Albertson, 2005; Inazawa et al, 2004; Albertson and Pinkel, 2003; Pollack et al, 2002) and disease progression (Gonzalez et al, 2005).

Despite the usefulness for copy number determination, array CGH is not suited to address the other types of genomic structural variations, most notably, inversions, translocations and other types of nucleic acid rearrangements. Tuzun et al (2005) attempt to address these limitations with an approach termed "fosmid paired-end mapping." This approach relies on the head-full mechanism of fosmid packaging to produce genomic DNA libraries with reasonably uniform ∼40 kb size genomic inserts from test subjects. End-terminal sequencing of the randomly selected ∼40 kb library inserts produces pairs of short sequence tags in which each tag-pair marks two genomic positions with separation of approximately 40 kb along the lengths of the target DNA. The tag-pairs are then computationally aligned to a reference genomic assembly and any discordance with either their expected orientation or with their ∼40 kb separation distance, would denote the presence of at least one structural difference between target and reference nucleic acid spanning that region. Tag-pairs having map positions that are separated by more than 40 kb signify the presence of a deletion on the target DNA in respect to the reference; map positions with separation of less than 40 kb signify an insertion of DNA in the target. Inconsistencies in the orientation of the pair of mapped tags denote potential DNA inversions or other complex chromosomal rearrangements. Chromosomal translocations are signified by assignment of the tag-pair to two different chromosomes on the reference sequence. Analysis of over a million fosmid clones enabled Tuzun et al (2005) to identify nearly 300 sites of structural variations between test subject and the reference genomic assembly.

While fosmid paired-end mapping is a useful start to identify fine-structural-variations in the Human Genome, the immense cost and logistical efforts required to purify and sequence more than a million fosmid insert ends for each test subject preclude its use in broad population and cohort surveys to identify genomic variations that could be associated with complex disease or in response to environmental factors and the like. Furthermore, fosmid vectors and their variants generally propagate in very low copy-numbers in host cells making reliable automated DNA production and sequencing difficult to maintain. Hence, there is a need for an efficient, robust high throughput and low cost method for the identification of fine-structural-variations for use in genomic and association studies to link these genetic elements to disease, disease progression and disease susceptibility. The present invention provides these and other substantial benefits.

### SUMMARY OF THE INVENTION

The present invention provides a method for nucleic acid mapping and identification of fine structural variations in nucleic acids and utilities, wherein two genomic variation tags (GVTs) are juxtaposed and the two constituent members of the tag pair (GVT-pair) are unique positional markers of a defined separation distance in a target nucleic acid molecule, the method comprising:
- fragmenting DNA of a target population either randomly or at defined sites and then purifying the fragmented DNA sample to a predetermined size;
- ligating a DNA adaptor having one or more restriction endonuclease recognition sites to both ends of a fragmented target DNA insert;
- ligating the target DNA linked adaptor to a circular modular vector;
- digesting the adaptor using a restriction endonuclease, which recognizes a restriction endonuclease binding site within the adaptor sequence, to cleave the target DNA insert at a defined distance from each end of the target DNA insert to create two sequence tags (GVTs) comprising terminal sequences of the target DNA insert that are attached to the plasmid vector; and
- recircularizing the plasmid vectors with the attached GVTs to obtain a circular plasmid including a GVT pair having two juxtaposed GVTs;
- excising the newly created GVT pair from the vector;
- producing oligomerized GVT pair monomers for efficient DNA sequencing.

The present invention also provides a method for nucleic acid mapping and identification of fine structural variations in nucleic acids and utilities, wherein two genomic variation tags (GVTs) are juxtaposed and the two constituent members of a tag pair are unique positional markers flanking two adjacent and cleavable restriction endonuclease sites, for one or more given restriction endonucleases, along the length of a population of target nucleic acid molecules, the method comprising:
- fragmenting DNA of a target population either randomly or at defined sites and then purifying the fragmented DNA sample to a predetermined size;
- ligating the digested target DNA inserts into a circular modular vector at a location flanked by a pair of sites for a type IIS, type IIG, or type III restriction endonuclease;
- cleaving the insert DNA at a defined distance from each end of the target DNA inserts, thereby creating two sequence tags (GVTs), comprising terminal sequences of the target DNA inserts that are attached to the vector backbones; and
- recircularizing the vector backbones with the attached GVTs to form a circular plasmid each bearing a GVT-pair comprising two juxtaposed GVTs;
- excising the newly created GVT pair from the vector;
- producing oligomerized GVT pair monomers for efficient DNA sequencing.

According to the present invention, DNA of a target population for analysis is fragmented either randomly or at defined sites. In certain embodiments, the fragmented DNA sample is purified to a predetermined size that defines a spatial window that sets the resolution level for analysis. To the ends of the fragmented DNA are attached a short synthetic DNA adaptor that comprises an appropriate cohesive overhang to facilitate cloning the adaptor-ligated sample DNA into a suitable vector. The adaptor incorporates a recognition site for a suitable type IIS, type IIG or type III restriction endonuclease (for example: *Mme* I, *Nme*A III, *Cst*M I, *Eco*P15 I, *Pst* II, *Hpy*790545P, or their preferred functional equivalent) in an orientation such that digestion of a library of insert-bearing plasmids with an aforementioned restriction endonuclease enzyme, cleaves the DNA inserts at a useful and defined distance from each insert terminus causing the release of the intervening sequence to yield a pair of Genomic Variation Tags (GVTs) that are attached to the vector. The newly linearized vector-GVT complexes are re-circularized by ligating the GVTs together to produce GVT-pairs that represent the two terminal regions of the original target DNA insert. Transfection of the circularized recombinant plasmids into host cells yield a primary GVT-pair library comprising individual plasmid clones each carrying a GVT-pair. The primary library is amplified and the purified plasmids are digested with a second restriction endonuclease that cuts at sites flanking the GVT pairs to release it from the plasmid vector. The released GVT-pairs are purified, oligomerized to a suitable size and are subcloned into a suitable vector for efficient high-throughput DNA sequence determination of the oligomerized GVT-pairs. When the sequence of individual GVTs of a GVT-pair are aligned computationally onto a reference sequence, any discordance with either their expected identity, separation distance or orientation with those aligned on the reference signals the optimized vector and host systems to facilitate efficient high-throughput sequence determination of GVT-pairs.
According to the present invention, DNA of a target population for analysis is fragmented either randomly or at defined sites. In certain embodiments, the fragmented DNA sample is purified to a predetermined size that defines a spatial window that sets the resolution level for analysis. To the ends of the fragmented DNA are attached a short synthetic DNA adaptor that comprises an appropriate cohesive overhang to facilitate cloning the adaptor-ligated sample DNA into a suitable vector. The adaptor incorporates a recognition site for a suitable type IIS. type IIG or type III restriction endonuclease (for example: Mme **I,** *NmeA* III, *CstM* I, *Eco*P15 I, *Pst* II, *Hpy*790545P, or their preferred functional equivalent) in an orientation such that digestion of a library of insert-bearing plasmids with an aforementioned restriction endonuclease enzyme, cleaves the DNA inserts at a useful and defined distance from each insert terminus causing the release of the intervening sequence to yield a pair of Genomic Variation Tags (GVTs) that are attached to the vector. The newly linearized vector-GVT complexes are re-circularized by ligating the GVTs together to produce GVT-pairs that represent the two terminal regions of the original target DNA insert. Transfection of the circularized recombinant plasmids into host cells yield a primary GVT-pair library comprising individual plasmid clones each carrying a GVT-pair The primary library is amplified and the purified plasmids are digested with a second restriction endonuclease that cuts at sites flanking the GVT pairs to release it from the plasmid vector. The released GVT-pairs are purified, oligomerized to a suitable size and are subcloned into a suitable vector for efficient high-throughput DNA sequence determination of the oligomerized GVT-pairs. When the sequence of individual GVTs of a GVT-pair are aligned computationally onto a reference sequence, any discordance with either their expected identity, separation distance or orientation with those aligned on the reference signals the presence of one or more fine structural differences between target and reference nucleic acids in the region spanned by the GVT-pair. Thus, the tabulated sequences of a plurality GVT-pairs constitute a detail genomic profile of the target nucleic acid population in respect to the reference sequence. These and other aspects of the invention will become evident upon reference to the following detailed description. In addition, various references (including patents, patent applications and journal articles) are identified below and are incorporated by reference herein.

Useful utilities offered by the present invention include but are not limited to the rapid construction of high-resolution genomic maps that can be used to: (1) identify fine-scale-variations of the genome that contribute to human diversity and might be causal to disease, disease progression or disease susceptibility and other observed traits for use as diagnostics or as targets for therapeutic intervention; (2) enable the design and creation of oligonucleotide microarray or other assay methods for rapid and massively parallel interrogation of fine-structural-variants in DNA samples for medical diagnosis, genotyping, and other such useful applications; (3) facilitate accurate and rapid DNA assembly from whole genome or shotgun DNA sequencing approaches; (4) identify fine-structural-variations of RNA transcripts resulting from differential RNA processing to aid genomic annotation, functional genomic studies, and potential disease diagnosis; (5) create genomic profiles to facilitate comparative genomics and phylogenic studies and to aid differential identification of closely related organisms; and (6) create genomic profiles of related strains, race, biotypes, variants, breeds or species to identify genomic elements that might be causal to any observable phenotypes of academic, medical or of commercial interest.

### PRIOR ART OF THE INVENTION

The following methods provide background for the practice of the present invention and extends and combines aspects of the prior art to yield the novel and improved methods described and for the utilities indicated.

### 1. FOSMID PAIRED-END MAPPING

Tuzun et al (2005) described the method of fosmid paired-end mapping where pairs of short sequence tags, separated by approximately 40 kb, were generated by terminal-end-sequencing of random ∼40 kb genomic inserts derived from human fosmid genomic libraries. Following alignment of tag-pairs to a reference genomic assembly, structural variations within the target DNA spanned by the tag-pairs were identified by discordance of expected marker separation distance and/or orientation with those aligned on a reference sequence. The method outlined by Tuzun et al (2005) relied on fosmid packaging to produce tag pairs of ∼40 kb (experimentally, the actual fragments range from 32 to 48 kb, < 3 standard deviations from the mean, 39.9 +/- 2.76 kb) separation distance on genomic DNA. The authors did not teach or disclose other methods to create tag-pairs, to create tag-pairs of different spacing to change the spatial resolution of analysis, to improve the homogeneity of the inert lengths in their library, nor did they teach or disclose methods to produce other types of sequence tag-pairs such as those of the present invention that can demarcate genomic positions based on the location and/or separation distance between pairs of adjacent endonuclease cleavage sites.

Many types of fine-structural-variations are not resolved by the ∼40 kb resolution window fixed by the fosmid-paired end mapping approach. Fosmid paired-end mapping has further limitations. Fosmid vectors propagate in host cells at very low copy numbers, a property used to minimize potential recombination, rearrangement and other artifacts encountered during the propagation of certain genomic sequences in a bacterial host. Despite the current use of amplifiable versions of fosmid vectors (Szybalski U.S. Patent No. 5, 874, 259) terminal sequencing of fosmid clones to generate tags still has very poor economy due to low DNA yield when compared to conventional plasmids, making high-throughput automated template production and sequencing difficult to maintain. Furthermore, two separate sequence reactions are required to generate a tag-pair sequence from a single fosmid DNA template, thereby reducing the economy further. The present invention overcomes these limitations through: (1) the ability to produce GVT-pairs whereby the spacing of tag-pair members on the target DNA can be engineered from 50 bp or less to several hundreds of kilo bp or more to tailor detection resolution to suit the analysis of different types of nucleic acids and to suit any given experimental design; (2) considerably more accurate and uniform spacing between tag-pair members for greater analytical precision; (3) the ability to produce genomic tag-pairs based on other criteria besides separation distance, such as the creation of tag-pairs based the location and/or the relative separation distance of adjacent endonuclease sites for improved interrogation of the target nucleic acid sample; and (4) oligomerization of GVT-pairs and subcloning the GVT-pair oligomers into a vector optimized for high-throughput DNA sequencing to reduce operational cost, thereby enabling the present invention for use in broad population and cohort studies.

### 2. METHODS FOR THE GENERATION OF GENOMIC TAGS

A variety of DNA-based fingerprinting approaches have been described in the art to characterize and to compare genomes (Wimmer et al, 2002; Kozdroj and van Elsas, 2001; Rouillard et al, 2001; Schloter et al, 2000). All these approaches employed some combinations of restriction digestion of the target DNA, PCR amplification, or gel electrophoretic separation. In common, these approaches are laboriously encumbered by the need to extract candidate DNA fragments from gels for DNA sequencing. A step forward was the recent work of Dunn et al (2002) where they described a method using the type IIS/type IIG restriction endonuclease, *Mme* I, to generate "Genomic Signature Tags" (GSTs) for analyzing genomic DNA (Dunn et al, 2002). GSTs were generated by ligation of adaptors bearing a Mme I recognition site to genomic DNA fragments that were initially created by an initial digestion of the target DNA with a type II restriction enzyme followed by a second digestion with a frequent cutting tagging enzyme. Digestion of the adaptor ligated DNA with *Mme* I created a 21-bp tag (GST) with a fixed position in the DNA relative to the sites recognized by the initial restriction enzyme digestions. Following amplification by PCR, purified GSTs were oligomerized for cloning and sequencing. The identity of the tags and their relative abundance were used to create a high-resolution "GST sequence profile" of genomic DNA that can be used to identify and quantify the genome of origin within a given complex DNA isolate. Using *Yersinia pestis* as a model system, Dunn et al (2002) were able to define areas in a relatively simple genome that might have undergone changes that added or deleted restriction sites. However, the method of Dunn et al (2002) has limited utility in complex genomes such as that of man, where many structural variations are not revealed by the simple gain or lost of a site for a small number of restriction endonucleases under investigation. Furthermore, the number of GSTs required to cover a large genome or to analyze multiple samples for even one restriction site is prohibitive. In contrast to the method of Dunn et al, the GVT-pairs of the present invention provide the economy and the analytical power to profile complex genomes or to extend analysis to multiple DNA samples.

Versions of a method known as Serial Analysis of Gene Expression (SAGE), first described by Velculesu et al (1995) and Kinzler et al (*U.S. Patent No*. *5,695,937*), also made use of a type IIS or a type IIG restriction endonuclease to generate DNA tags (Ng et al, 2005; Wei et al, 2004; Saha et al, 2002). The so termed "SAGE tags" were generated from cDNA templates to provide an assessment of the complexity and relative abundance of cDNA species in a biological sample. Later versions of SAGE referred to as "LongSAGE" made use of *Mme* I digestion to create tags of 21-bp in length to tag mRNA transcripts (Saha et al, 2002). The most current refinement termed "SuperSAGE" made use of the type III restriction endonuclease, *Eco*P15 I, to produce a longer tag of 26-bp for improved mRNA assignment to the genome (Matsumura et al, 2003). Although the present invention also makes use of type IIS, type IIG or type III restriction endonucleases to generate sequence tags, the resulting GVT-pairs of the present invention are fundamentally distinct from the aforementioned SAGE and GST tags by methods of production as well as by improved informational content. The use of pairs of linked tags offers a marked improvement in efficiency and analytical power over the use of unlinked tags for the generation of high-resolution physical maps that are particularly useful for characterizing novel genomes or annotating genomes and DNA samples for fine-structural-variations.

The recent work of Ng et al (2005) described a further development of the SAGE method. The investigators made use of a method pioneered by Collins and Weissman (1984) where circularization of DNA fragments, also referred to as intra-molecular DNA ligation, was employed to link distal DNA segments together into a vector to produce the so termed "genomic jumping libraries" (Collins et al, 1987). Ng et al (2005) circularized individual cDNAs to link their 5'- and 3'- derived SAGE tags together to produce "Paired-End Ditags" (PETs), which are then oligomerized to facilitate efficient sequencing. PETs are useful for genomic annotation by the identification of transcription start sites and polyadenylation sites of transcription units to set gene boundaries and to aid the identification of their flanking regulatory sequences. While the production of GVT-pairs of the present invention and the production of PETs by the method of Ng et al (2005) both rely on intra-molecular ligation to achieve linkage of DNA markers, only the GVT-pairs of the present invention integrate accurate physical distance and other useful information between DNA markers thereby making GVT-pairs useful for detailed genomic structural analysis.
WO 2005/042781 A2 describes a method for producing a paired tag from a nucleic acid sequence in which the paired tag comprises the 5'end and 3' end tag of the nucleic acid sequence. In one embodiment, the nucleic acid sequence compises two restriction endonuclease recognition sites specific for a restriction endonuclease that cleaves the nucleic acid sequence distally to the restriction endonuclease recognition sites. In another embodiment, the nucleic acid sequence frther comprises restriction endonuclease recognition sites specific for a rare cutting restriction endonuclease.
WO 03/074734 A2 discloses methods for determining genome-wide sequence ariations associated with phenotype of a species in a hypothesis-free manner. In the methods of the invention, a set of restriction fragments for each of a subpopulation of individuals having the phenotype are generated by digesting nucleic acids from the individual using one or more different restriction enzymes. A set of restriction sequence tags for the individual is then determined from the set of restriction fragments.
EP 1 533 386 A2 teaches methods for gene identification signature (GIS) analysis. The invention utilizes an isolated oligonucleotide comprising at least one ditag, wherein the first tag comprises the 5'terminus sequence and the second tag comprises the 3'terminus sequence of a nucleic acid molecule or a fragment thereof. The ditag analysis is then used for gene discovery and genome maping.
The scientists of WO 2004/050918 A1 developed a method of providing an indication of an instance of expression of a gene. The method comprising the steps of providing a cDNA having a terminus comprising a terminal transcribed sequence of a gene, linking the cDNA to a linker sequence , cleaving the linked nucleic acid with the first nucleic acid cleavage enzyme to provide a linked tag, and detecting the presence or identity of the linked tag or the nucleotide sequence tag to provide an indication of an instance of gene expression.
None of these methods, however, teaches to create tag-pairs of defined spatial spacing or of other criteria, nor did they describe how structural variations such as those that arise from mRNA processing or fine-structural-variations in the genome can be derived using their approaches.

### 3. MULTIPLEX SEQUENCING VECTOR

As used herein, the term "multiplex sequencing vector" refers to a plasmid vector optimized for high-throughput Sanger dideoxy sequencing that has the capacity to carry two or more independent inserts resulting in a plurality of sequencing reads from a single template, thereby enjoying cost saving through the economical use of materials.

The art as it is generally practiced is that one plasmid vector propagates a single DNA insert. Typical of such a configuration, a plasmid template can produce two sequencing reads from each of the two vector primer-binding sites flanking the DNA insert. Mead and Godiska (U.S. Patent No. 6, 709, 861*)* described a "multiplex cloning vector" whereby DNA inserts are cloned into dispersed sites of a cloning vector, thereby allowing insert sequences to be subsequently sequenced either simultaneously in a single DNA sequencing reaction, or in parallel reactions using the same template preparation.

The multiplex cloning vector described by Mead and Godiska is available commercially as *pLEXX-AK* (Lucigen Corporation, Middleton, WI), and it is the principal component of the CLONEPLEX™ library construction system. Plasmid vector, *pLEXX-AK,* is provided by the vendor as two dephosphorylated blunt-ended vector DNA segments. Each vector segment carries a separate drug selectable marker and a pair of sequencing primer-binding sites for DNA sequencing. The vector system was promoted to reduce material cost for high throughput sequencing applications. In actual practice, the major high throughput application for DNA sequencing is shotgun genomic sequencing to which the *pLEXX-AK* vector system is not particularly well suited. In principle, the addition of phosphorylated blunt-end DNA inserts to a ligation reaction containing the two dephosphorylated *pLEXX-AK* vector segments would produce a configuration where a DNA insert is ligated between each of the two vector segments to yield a functional circular molecule. In practice, a complex milieu of ligation products is actually produced, in which only a small portion of the products comprises the desired circular molecule whereby a single DNA insert is ligated between the two different vector segments. While drug resistant markers on each of the two vector segments allow the selection of the productive species from the milieu, the system is inherently inefficient due to random undirected blunt-end ligation of the constituent vector and insert fragments. A large proportion of the input DNA inserts are expended in non-productive ligation events and a relatively large amount of starting DNA is needed to offset the lost. Most critically, the absolute requirement for phosphorylated blunt-end DNA inserts for cloning into the two sites of *pLEXX-AK* places a severe constraint on applications where sequence continuity of the original DNA inserts is critical such as for the construction of genomic DNA libraries for shotgun sequencing. For this application, any genomic insert ligated to other genomic insert (the so called chimeric inserts) during library construction would severely undermine the subsequent genomic assembly constructed from the sequence data. Furthermore, despite the claim by the investigators that their approach could be extended to the construction of vectors bearing independent inserts at three or more dispersed sites on the vector to increase efficiency further, the reliance on blunt-end ligation and the need for multiple selection markers for retention of each vector segments makes the claim impractical to carry out in practice.

The present invention overcomes the aforementioned limitations of the approach described by Mead and Godiska (U.S. Patent No. 6,709,861*)* for the construction of a multiplex sequencing vector and provides improved materials, methods, and strategies for directed assembly of ever-more complex DNA molecules, vector and vector components to facilitate efficient multiplex DNA sequencing and other applications. Specifically, the present invention describes a modular vector system whereby individual vector components are flanked by unique type IIS restriction enzyme sites to create asymmetric cohesive ends to direct the ordered assembly of the vector modules and intervening DNA elements to any desired configuration at high efficiency to acquire new functionalities. A plasmid derived from the present invention, *pSLGVT-3,* is a high number copy plasmid optimized for high-throughput DNA sequencing and can carry at least two independent inserts to enable four separate sequencing reads from a single template. A second plasmid, *pSLGVT-2,* is a low copy number plasmid variant of *pSLGVT-3* that is optimized for propagation of long DNA segments or those inserts that might be difficult to propagate in a microbial host without rearrangement or recombination. The two independent cloning sites on *pSLGVT-2* and *pSLGVT-3* make use of unique sets of non asymmetric complementary cohesive ends for the ordered and specific ligation of independent inserts at the two cloning sites, thereby abrogating the need for blunt-end cloning and the requirement for phosphorylated DNA inserts the principle cause for the generation of insert chimeras during library construction. Another distinguishing feature *pSLGVT-*series of plasmids from *pLEXX-AK* of Mead and Godiska (U. S. Patent No. 6, 709, 861*)* is the use of the plasmid replicon as a biological selection of correct plasmid assembly, thereby reducing the material size of the vectors to increase the insert size carrying capacity. If required, the modular construction of the *pSLGVT* vectors and the use of asymmetric cohesive ends between vector modules permit rapid reconfiguration of the vector system to carry three or more independent DNA inserts.

### DETAILED DESCRIPTION OF THE INVENTION

### 1. PREPARATION AND FRAGMENTATION OF NUCLEIC ACIDS FOR PRODUCTION OF GVT-PAIRS

As described herein, the present invention provides methods to produce high-resolution genomic maps that can be used to characterize unknown genomes or to identify fine-structural-variations between target populations of nucleic acids and a reference sequence. Target nucleic acids suitable for analysis include but are not limited to: genomic DNA of eukaryotic and prokaryotic organisms, microbial DNA, plastid DNA, plasmid and phagemid DNA, viral DNA and RNA, complementary DNA (cDNA) derived from ribonucleic (RNA), and DNA produced by *in vitro* amplification such as by PCR among others. Methods for DNA isolation from aforementioned sources, synthesis of cDNA from RNA and for the amplification of nucleic acids are known to those skilled in the art.

For certain embodiments of the present invention, the genomic distance spanned by the GVT-pair determines the resolution level for analysis. The smaller the spacing between GVTs, the higher is the resulting spatial resolution for mapping and for detecting fine-structural-variations in a target population of nucleic acid. Large GVT spacing requires fewer GVT-pairs to cover a DNA sample of a given complexity but with a concomitant decrease in spatial resolution. For identification of mRNA processing variants, GVT- spacing of 50 or 100 bp offers sufficient resolution levels to detect most products of alternative splicing in cDNA populations. For human whole genome surveys, GVT spacing of 10, 25, 50 or 100 kb offers a productive compromise between resolution and economy. The functional tradeoff between GVT spacing, the resolution level required to detect different types of DNA structural variations, and the number of GVT-pairs needed to cover a given sequence complexity to a required depth can be modeled computationally to derive an optimal experimental design for a given application.

As described above, the material length of target DNA insert used for the construction of the GVT-pairs governs the separation distance between resident GVTs of a GVT-pair, thus setting the resolution level for the analysis. Methods to create and to purify a near size-homogeneous population of fragmented nucleic acids are described in the art. Fragmentation of target DNA to a desired length can be accomplished enzymatically under conditions of partial or complete digestion with a variety of restriction endonucleases. The use of restriction endonuclease with recognition sites of six or greater base pairs are useful to produce longer DNA fragments. The use of frequent cutting type II endonucleases such as *Mbo* I, *Hae* III, and the like, which cut DNA once on average every 256-bp, is known in the art for producing varied sizes of DNA fragments by partial digestion. The use of restriction endonuclease CviJ I under relaxed conditions, which cleaves DNA at GC dinucleotide positions (Fitzgerald et al, 1992), is particularly useful under partial digestion conditions to produce a useful continuum of DNA fragment sizes. In some embodiments, randomly generated DNA fragments are useful. The method for random generation of DNA fragments include: (1) digestion with bovine pancreatic deoxyribonucleic acid nuclease I (DNase I), which makes random double-strand cleavages in DNA in the presence of manganese ions (Melgar and Goldthwait, 1968; Heffron et al, 1978); (2) physical shearing (Shriefer et al, 1990); and (3) sonication (Deininger, 1983).

Conditions for partial enzymatic digestion are determined empirically, varying one or more parameters of reaction volume, enzyme concentration, and enzyme to substrate ratio, incubation time or temperatures. For high-resolution analysis requiring a GVT separation of ∼ 5 kb or less, fragmentation methods that are not sequence dependent is preferred. The HydroShear™ instrument (Genomic Solutions Inc, Ann Arbor, MI) is particularly useful for generating random DNA fragments of a defined size range. Random DNA fragments can also be generated through the use of random primers during cDNA synthesis or by use of PCR, alone or in combination with the other fragmentation methods described. The progress of fragmentation to yield the desired length product is most easily monitored by gel electrophoresis. Following generation of a suitable DNA size-distribution, T₄ DNA polymerase is used to repair or to make blunt the DNA ends in preparation for blunt-end ligation to GVT-adaptors for the production of the GVT-pairs of the present invention. In cases where DNA is fragmented by partial or complete digestion with one or more endonucleases leaving cohesive ends, repair is not necessary but the design of the GVT-adaptor will need to accommodate the cohesive ends generated by the fragmentation enzyme. Since ligation of inserts to other inserts destroys the co-linearity of the target DNA and undermines the construction of the genomic map, the insert DNA's 5' phosphate groups are removed by a phosphatase to prevent the ligation of insert DNA to other insert DNA during ligation to GVT-adaptors.

### 2. SIZE FRACTIONATION AND PURIFICATION OF SIZE-SELECTED DNA

For certain embodiments, dephosphorylated DNA inserts are fractionated by gel electrophoresis and are purified to yield DNA inserts of a desired size. Poly-acrylamide gels are best used for fractionation of DNA from 50 bp to 1 kb. For fragment sizes of -250 bp to 20 kb, 0.4% to 3% agarose gels are suitable. Pulsed field gel electrophoresis is suitable for fractionating DNA from ∼10 kb to several hundreds of kb in size. These procedures are described in references therein (Birren and Lai, In: Pulse Field Electrophoresis: A Practical Guide, Academic Press, San Diego, 1993; Rickwood and Hames (eds), In: Gel Electrophoresis of Nucleic Acids: A Practical Approach, Oxford University Press, New York, 1990; Hamelin and Yelle, 1990). DNA is sized with the use of suitable size markers electrophoresed in parallel with the sample and are visualized by staining. Gel slices containing DNA of a desired size are excised with a scalpel, where after the DNA is recovered from the gel matrix by electro-elution or by enzymatic or chemical degradation of the gel matrix. The recovered DNA fragments for analysis should be near homogeneous in size. Gel systems and electrophoretic conditions for maximizing separation resolution are known in the art. Two or more cycles of gel electrophoresis are used to obtain greater sample size homogeneity. Sample with size variance of more than 2.5% from the mean length may contribute to unacceptable noise for use by the present invention.

### 3. DESIGN OF GVT-ADAPTOR AND LIGATION TO TARGET DNA

Those skilled in the art would realize the existence of a plurality of GVT-adaptor designs suitable for use in the present invention. In sum, a suitable GVT-adaptor comprises the following material properties: (1) a short top strand and a short bottom strand of 5' phosphorylated oligonucleotides of unequal lengths capable of stable complementary base-pairing to yield a.two strand structure; (2) one end of the GVT-adaptor has a short non palindromic single strand protrusion that can ligate to a vector having the complementary sequence; (3) the other adaptor end has a blunt-end structure or other suitable end structures to enable efficient ligation to dephosphorylated target DNA fragments; (4) the end of the adaptor that flank target DNA bears a suitable type IIS, type IIG or type III restriction endonuclease recognition site in an orientation such that the site directs cleavage at a fixed and useful distance on the target DNA to produce the GVT; and (5) adjacent or overlapping the type IIS, type IIG or type III enzyme recognition site is a second restriction endonuclease site for excising the created GVT-pair from the vector. Illustrative examples of suitable GVT adaptors are depicted below (examples Nos. 1-4).

### EXAMPLE NO 1: GVT (Mme I)-adaptor for blunt ligation to dephosphorylated target DNA.

The sequence 5'pGACA-3' of illustrative example no. 1 (SEQ ID NO 1), is a cohesive end for sub cloning adaptor-ligated DNA insert into a vector with a pair of protruding 5'-TGTC-3' sequence. The cohesive end is non-palindromic to prevent the formation of adaptor dimers and multimers of DNA bearing ligated adaptor and prevents the creation of insert-less vectors. The 5'-CAGAGGA -3' sequence of SEQ ID NO 1 and its reverse complement, 5'-TCCTCTG-3', of SEQ ID NO 2 depict a short sequence capable of stable complementary base pairing to aid the formation of a functional two-strand adaptor. The 5'-TCCAAC-3' sequence of SEQ ID NO 1 and its reverse complement, 5'-GTTGGA-3', of SEQ ID NO 2 is the recognition site for the type IIS endonuclease, *Mme* I (Boyd et al, 1986). *Mme* I cleaves DNA 20 bp downstream (that is in a 5' to 3' direction) from its 5'-TCCAAC-3' recognition site and 18 bp upstream (that is in a 3' to 5' direction) from its reverse complement on the opposite strand to yield a 20-bp GVT, with 2-bp a protruding 3'-overhang. Overlapping the *Mme* I recognition site is the recognition site for *Bam*H I, 5'-GAATTC-3'. *Bam*H I cleavage serves to release of the created GVT-pair from the vector. The *Bam*H I site overlaps the *Mme* I site in order to minimize extraneous adaptor sequences for greater economy during sequence determination of the oligomerized GVT-pairs. To achieve the same end in other adaptor designs, an overlapping *Bsp*T I site can be used for the excision of GVT-pairs that are created by *CstM* I digestion. Similarly, *Kas* I can be used to excised GVT-pair created through the digestion with *NmeA* III.

### EXAMPLE NO 2: GVT (Mme I)-adaptor ligation to dephosphorylated target DNA digested with Xba I.

The salient features of the GVT (*Mme* I)-adaptor of illustrative example No. 2 is identical to those of illustrative example No. 1, with the added incorporation of a 5'-pCTAG-3' overhang (SEQ ID NO 3) to direct ligation of the adaptor to *Xba* I digested dephosphorylated target DNA fragments. Those that are skilled in the art would realize that the adaptor of example No. 2 is but one variant. There exist other functional adaptor variants created through the incorporation of a suitable overhang that ligate to target DNA digested with other restriction endonucleases to suit different experimental designs.

### EXMAPLE NO 3: GVT (EcoP15 I)-adaptor for blunt ligation to dephosphorylated target DNA.

### EXAMPLE NO 4: GVT (EcoP15 I)-adaptor for cohesive-end ligation to dephosphorylated target DNA digested with Xba I.

Illustrative example Nos. 3 and 4 depict adaptor designs that utilize the type III restriction endonuclease, *Eco*P15 I, to produce a 27-bp GVT. A restriction endonuclease site for *Pst* I (5'-CTGCAG-3') for excision of the GVT-pair overlaps the *Eco*P15 I site (5'-CAGCAG-3'). Overlapping the *Pst* I site with the *Eco*P15 I site minimizes extraneous adaptor sequence within GVT-pair for greater economy during sequencing. The adaptor of illustrative example No. 4 incorporates a *Xba* I cohesive end to direct ligation of adaptor to dephosphorylated *Xba* I digested target DNA fragments. Those that are skilled in the art would realize that the adaptor of example No. 4 is but one variant. There exist other functional adaptor variants created through the incorporation of a suitable overhang that ligate to target DNA digested with other restriction endonucleases to suit different experimental designs.

The illustrative GVT-adaptors of illustrative example Nos. 1 and 2 can either produce an 18 bp or 20-bp long GVT by digestion with *Mme* I. An 18-bp GVT is produce when T₄ DNA polymerase is used to remove the 3'-overhang created from *Mme* I cleavage prior to blunt-end ligation of the linked GVTs to generate a 36-bp GVT-pair. A 20-bp GVT results when an adaptor having 16-fold degenerate 5'-overhangs, compatible with all possible two-base 3' overhangs generated from *Mme* I digestion, is used to ligate the GVTs together to produce the GVT-pair. In contrast to *Mme* I, *Eco*P15 I cleavage creates 2-bp 3' recessed ends, which is extended by DNA polymerase to yield a 27-bp blunt-ended GVT, from which a 54-bp GVT-pair is created by blunt-end ligation.

Any type IIS or type IIG restriction endonucleases that recognize an uninterrupted nucleotide sequence and cleaves at least ten base pairs distant from its recognition site are suitable for use in the generation of GVT. These enzymes include: *BceA* I*, Bpm* I*, BpuE* I, *Bsg* I, *BsmF* I, *Bst*V1 I, *Eco*57 I, *Eco*57M I, *Gsu* I, *CstM* I, *NmeA* III, and *Mme* I. Of these, *Mme* I, *NmeA* III, or *CstM* I are preferred for use by the present invention since their cleavage site is the most distant from its DNA recognition site among the type IIS endonucleases described to date, thereby producing a GVT of the longest length. It is anticipated that other type IIS or type IIG endonucleases with longer defined cleavage distance from its recognition site will be discovered in the future and can be used by the present invention. For reviews of the type IIS and IIG restriction endonucleases see Sistla and Rao (2004) and Bujnick (2001).

The type III restriction endonucleases were initially described to require two inverted asymmetric recognition sites and that cleavage in vivo occurs distal to only one of the two pairs of inverted recognition sites chosen at random. For review, see Sistla and Rao (2004) and Bujnick (2001). Such properties are not useful by the present invention. However, characterization of the prototype type III enzyme, *Eco*P15 I, indicated that recombinant or the purified native enzyme when used at two to three-fold higher concentration and in the presence of potassium ions is capable of promiscuous cleavage at single sites *in vitro* (Sistla and Rao, 2004; Raghavendra and Rao, 2004; Peakman et al, 2003; Mucke et al, 2001). This newly described property of *Eco*P15 I was exploited to produce SAGE tags from cDNA (Matsumura et al, 2003). The *Eco*P15 I enzyme is commercially available (New England Biolabs, Ipswich, MA) and is used by the present invention to produce a 27-bp GVT and a subsequent 54-bp GVT-pair. Other type III endonucleases that cleave DNA at a useful distance from its recognition site can be used by the present invention.

Those of skill in the art know methods for ligation of adaptor to DNA insert and for general ligation of nucleic acid molecules. See, for example, Ausubel et al (eds) (In: Short Protocols in Molecular Biology, 3rd Ed, John Wiley & Sons, New York, 1995). Typical ligation conditions for blunt-end liagtion of adap- ' tor to DNA insert call for a -50 to 500-fold molar excess adaptor to target DNA, high T₄ ligase concentration, or the inclusion of a volume exclusion agent such as polyethylene glycol (Hayashi et al, 1986; Pheiffer and Zimmerman, 1983; Zimmerman and Pheiffer, 1983). Liagtion of adaptor to cohesive end target DNA requires ∼5-fold molar excess. GVT-adaptor-ligated DNA inserts are passed through a ChromoSpin™ column (Clontech, Mountain View, CA) to remove excess adaptors before purification and size-selection by gel electrophoresis. To generate GVT-pairs by intra-molecular ligation, the purified products are ligated into one of several plasmid vectors described below.

### 4. VECTOR CONSTRUCTION FOR GVT-PAIR PRODUCTION

An aspect of the present invention provides general methods to produce cloning vectors that are capable to generate GVT-pairs by digestion of the insert with either a type IIS, type IIG or a type III endonuclease followed by intra-molecular ligation. A GVT-DNA cloning cassette comprising the material features depicted below is used to modify existing cloning vectors by ligation of the cassette into a suitable site.

### EXAMPLE NO 5: Illustrative example of a GVT-DNA cloning cassette.

The GVT-DNA cassette is produced synthetically from two complementary oligonucleotides (SEQ ID NO 7 and SEQ ID NO 8) annealed to form a double strand structure with terminal cohesive ends suitable for ligation into an existing vector. As an illustrative example, the DNA cassette above is shown with protruding cohesive ends for *Eco*R I for replacement of the multiple cloning site region of *pSMART VC* or *pSMART-cDNA* (Lucigen, Middleton, WI) to render these plasmid vectors capable to produce GVT-pairs in conjunction with the previously described GVT-adaptor ligated target DNA. A material feature of the DNA cloning cassette is an inverted pair of type IIS or type IIG endonuclease sites; *Esp3* I in the illustrative example. Digestion of modified vector with *Esp3* I creates a pair of non-palindromic overhangs (5'-TGTC-3') on the vector that ligate specifically to complementary overhangs (5'-GACA-3') extending from GVT-adaptor-ligated target DNA inserts. The non-rotational symmetry of the vector and insert overhangs essentially eliminates the creation of insert-less plasmids and plasmids bearing multiple copies of adaptor-ligated target DNAs, which would compromise the colinearity of the target DNA insert and the subsequent creation of the genomic profile. The DNA cassette also incorporates the restriction endonuclease site, *Xba* I, situated between the pairs of inverted *Esp*3 I sites. A "stuffer DNA" fragment of a suitable size cloned at the *Xba* I site enables the monitoring of *Esp*3 I digestion during vector preparation. The length of the stuffer DNA fragment is selected such that *Esp*3 I single-digested, double-digested and undigested vector species can be easily resolved by gel electrophoresis and only fragments from double digestion are purified for use.

Those that are skilled in the art would realize that as with the previously described examples of suitable GVT-adaptor, the DNA cloning cassette described above is but one of a plurality of functionally equivalent designs. For example, the *Esp*3 I sites in the DNA cassette can be substituted with those of other type IIS or type IIG endonucleases where DNA cleavage is distal from a contiguous recognition site. Suitable type IIS or type IIG enzymes include: *Alw* I*, A*/*w*26 I, *Asu*HP I, *Bbv* I *, Bcc* I, *Bse*G I, *Bse*Mi I, *BsmA* I. *Bsm*F I, *Bso*MA I, *Bsp*CN I, *Bsp*M I, *Bsp*P I, *Bsp*TN I, *Bst*F5 I, *BstV1* I, *Fau I, Fok* I*, Hga* I, *Hph* I*, Lwe* I, *Ple* I*, Pps* I*, Sfa* I, *Smu* I, *Tsp*DT I, *Tsp*GW I, *Bbs* I, *Bci*V I, *Bfi* I*, Bfu,* I, *Bmr* I*, Bpi* I*, Bpm* I*, Bpu*A I*, Bpu*E I*, Bsa* I*, Bse*3D I, *BseM* I, *Bse*R I, *BseX* I, *Bsg* I*, BsmF* I, *Bso*31I, *BsrD* I, *Eco*31 I, *Esp3* I, *BsfV2* I*, Bve* I*, Eam*1104 I, *Eci* I*, Eco*57 I, *Eco*57M I, *Faq I, Gsu* I, *Ksp*632 I, *Cst*M I, *Mme* I, *Nme*A III, *Taq* II, *Sap* I. their isoschizomers and other examples described by Szybalski et al (1991). Prefer enzymes are those with six base pair or longer recognition sites, (for example: *Bsp*M I, *Eco*31 I, *Esp*3 I, *Sap* I and their isoschizomers) since the sites for these enzymes are less likely to occur in vector backbones and reduce the need for site-directed mutagenesis to eliminate these sites during vector construction. Also obvious to those that are skilled in the art are the precise sequences of the cohesive ends generated by the aforementioned enzymes can vary as long as they can form functional and specific base-pair with their intended ligation partners. The end structures on the DNA cassette can be modified to accommodate ligation of the cassette into the desired sites on preexisting vectors or to isolated vector components to create new vectors that can be used by the present invention.

The ability to propagate DNA segments stably in host cell is of critical importance for genomic analysis. Rearrangement or the lost of DNA segments containing AT- or GC- rich regions, repeats, hairpins, strong promoters, toxic genes and other problem sequences when propagated in host cell are of great concern for the study of fine-genomic-variations. DNA rearrangements and other cloning artifacts can be mistaken for structural variations in the target nucleic acid. Moreover, cloning bias can limit the size of inserts and can under-represent important regions of the genome from study. This problem was addressed recently by the development of fosmid and BAC vectors with conditional amplification systems (Szybalski U. S. Patent No. 5, 874, 259) where propagation of DNA is kept at one to two copies per host cell until induced to higher levels for analysis. Improved stability of genomic inserts of 15 kb to over 100 kb was reported and conditional amplification vectors are now in routine use for genomics studies. Conditional amplification fosmid/BAC vectors such as *pCC1FOS* (Epicentre, Madison, WI) and *pSMART VC* (Lucigen, Middleton, WI) and their variants are suitable for use in GST-pair production of GVT-spacing from 10 kb to 200 kb. However, use of conventional low-copy plasmid vectors appeared to be sufficient for stable maintenance of large DNA fragments without the need of BAC, PAC or fosmid type vectors (Feng et al, 2002; Tao and Zhang, 1998). The *pSMART* series of vectors offers low copy number propagation and has the added feature of having transcription terminators on the vector to reduce the potential effects of transcriptional interference, which might further improve DNA stability (Mead and Godiska U.S. Patent No. 6,709,861). For GVT-pair production of GVT-spacing from 50 bp to 10 kb or more, a variety of established and widely used low copy plasmid-based vectors are suitable for modification to produce GVT-pairs, including: *pBR322* (Bolivar et al, 1977) and *pACYC177* (Chang and Cohen, 1978).

Vectors for GVT-pair production are produced by insertion of the GVT-DNA cassette into a suitable vector backbone at suitable cloning site. General methods for ligating nucleic acid molecules are known to those of skill in the art. See, for example, Ausubel et al (eds) (In: Short Protocols in Molecular Biology, 3rd Ed, John Wiley & Sons, New York, 1995). For use, the vector backbone must be rendered free of the recognition sites for: (1) the type II, IIS or type IIG restriction endonuclease used to generate the cohesive ends on the DNA cloning cassette for direct cloning the target DNA or the adaptor-ligated target DNA; (2) the type IIS, type IIG or type III endonuclease used to generate the GVT from the cloned target DNA insert; and (3) the enzyme used to excise the newly created GVT-pair from the plasmid. For the illustrative examples of a GVT-DNA cassette and GVT-adaptors, the vector backbone needs to be free of a specific combinations of *Esp*3 I, *Eco*31 I, CstM I*, Mme* I*, Nme*A III, *Pst* II, *Eco*P15 I, *Bam*H I, Pst I, *BspT* I or *Kas* I sites, with the actual requirement dictated on the precise configuration of GVT-DNA cassette and adaptor in use. If required, the vector backbone can be rendered free of those aforementioned sites by site directed mutagenesis employing standard methods. See, for example: McPherson (ed) (In: Directed Mutagenesis: A Practical Approach, Oxford University Press, New York, 1991) and Lok (U.S. Patent No. 6,730,500). Typically, a substantial portion of vector DNA can be altered by single base-pair change to eliminate unwanted restriction endonuclease recognition sites without undue effects on vector functionality. Within protein coding sequences, single nucleotide changes are targeted to the codon wobble positions to maintain native protein coding. Changes made elsewhere on the vector backbone would require functional validation before use.

### 5. GVT-PAIR PRODUCTION VECTORS pSLGVT-1 AND pSLGVT-2

Plasmids, *pSLGVT-1* and *pSLGVT-2,* of the present invention are optimized and versatile vectors specifically designed to produce GVT and GVT-pairs employing *Mme* I or *Eco*P15 I, respectively, *pSLGVT-1* and *pSLGVT-2* are also free of *Cst*M I and *NmeA* III sites and can be used to produce GVT and GVT-pairs employing these two enzymes in accordance to the methods of the present invention. The basic vector comprises two chemically synthesized DNA modules to provide the basic maintenance functions of drug selection and plasmid replication, respectively. Connecting the two DNA modules to yield a circular molecule are DNA cassettes that provides specific utilitarian functions to the basic plasmid backbone. The vector modules bear terminal unique type IIS restriction endonuclease sites that create unique asymmetric cohesive ends to allow rapid future reconfiguration of the vector components to add or substitute modules or DNA cassettes for new functionalities.

] The first vector module comprises a modified *P15A* origin of replication. Plasmids bearing the *P15A* replicon propagate at a low number of approximately 15 copies per host cell (Sambrook et al (eds), In: Molecular Cloning: A Laboratory Manual, 2nd Ed, CSH Laboratory Press, Cold Spring Harbor, New York, 1989), thereby optimizing the stability of cloned genomic inserts. In contrast, high copy number plasmids, such as the *pUC* series of plasmids or *pBluescript,* may reach several thousand copies per cell. Two *Mme* I sites within the *P15A* replicon are each eliminated by a single nucleotide change to yield the *"P15A-m* replicon module" for the construction of plasmid *pSLGVT-1.* Mutation of these two sites is not expected to alter the secondary structure or the transcription of RNA II or RNA I required for the regulation of plasmid replication. The single *Eco*P15 I site in the *P15A* replicon is eliminated in the same fashion to yield the *"P15A-e* module" for the construction of plasmid *pSLGVT-2.* Both versions of the *p15A* modules are flanked at the RNA II promoter end of the module by a unique *Bpi* I site generating a 5' GTGA-overhang to facilitate ligation of DNA cassettes. For the same purpose, the replication fork end of the replication modules are flanked by *Faq* I site generating a 5 TCTC-overhang.

The second vector module comprises a modified version of the *Kan* gene from transposon *Tn903* conferring resistance to antibiotic Kanamycin (Grindley et al, 1980). Taking advantage of the wobble position and conforming to the optimal codon usage in *E*. *coli* whenever it is possible, four *Mme* I sites along with two *Nci* land *Nsi* I sites, and single sites for *Esp*3 I, *Pst* II, and *Hind* III are removed within the coding region of the *Kan* gene to yield the *"Kan* module". The *Kan* module is flanked at the *Kan* promoter end of the module by a unique *Sap* I site to generate a 5' TTG-overhang for DNA cassette ligation. The unique *BspM* I at the other end of the Kan module generates a 5' ACTG-overhang for the same purpose. Kanamycin drug selection is generally acknowledged to offer the best stability for the maintenance of plasmids bearing particularly long and/or difficult inserts and in many situations its use would also allow limited but convenient amplification of plasmid libraries in liquid cultures without undue clonal selection that can distort the composition of the plasmid library.

The core components of the *pSLGVT* series of plasmids are two DNA Cloning Cassettes, which provide specific insert cloning functionalities and serve to link the *Kan* module and the replicon modules together to yield a circular plasmid. Plasmids, *pSLGVT-1,* -2 and -3 have a common structure comprising the following material features on a circular map in the clockwise direction: (1) the Replicon Module; (2) DNA Cloning Cassette 1; (3) the *Kan* Module; and (4) DNA Cloning Cassette 2. Plasmid replication and the transcription of the *Kan* gene proceed in a clockwise direction. The structure of DNA Cloning Cassettes 1 and 2 is indicated below:

### EXAMPLE NO 6: DNA Cloning Cassette 1 and 2.

### DNA Cloning Cassette 1:

### DNA Cloning Cassette 2:

(GGTCTC)CTCAG( M13R) (SEQ ID NO:11)
(CCAGAG)GAGTC(<M13R)CACT (SEQ ID NO:12)
   *Eco31* I
T7 sequencing primer: 5'- TAA TAC GAC TCA CTA TAG GG-3' (SEQ ID NO:13)
T3 sequencing primer: 5'-ATTAACCCTCACTAA AGG GA-3' (SEQ ID NO:14)
M13 F sequencing primer: 5'-CAC GAC GTT GTA AAA CGA C-3' (SEQ ID NO: 15)
M13 R sequencing primer: 5'- GGA TAA CAA TTT CAC ACA GG-3' (SEQ ID NO: 16)

DNA Cloning Cassette 1 is produced from two complementary chemically synthesized oligonucleotides annealed to form a double strand structure with two terminal asymmetric 5' protruding cohesive ends, 5'-GAGA-3' and 5'-AAC-3', for directed ligation of the cassette to the 5'-TCTC-3' overhangs of the replicon module *(P15A-m* or *P15-e)* and to the 5'-GTT-3' overhang of the *Kan* modules, respectively. The binding sites on the DNA cloning cassettes 1 and 2 for sequencing primers T7, T3, M13 forward and M13 reverse are shown. Those that are skilled in the art would know of other sequencing primer binding sites suitable for use by the present invention. A pair of inverted *Esp*3 I sites on DNA cloning cassette 1 produces a pair of 5'-TGTC-3' overhangs on the vector to receive the GVT-adaptor ligated target DNA for the product of GVT-pairs. A *Xba* I site is situated between the set of *Esp*3 I sites for cloning a stuffer DNA fragment to help monitor the progress of *Esp*3 I digestion in the preparation of the vector to receive GVT-adaptor ligated target DNA. Flanking the *Esp*3 I sites are primer-binding sites for the T7 and T3 sequencing primers. These primers sites are used to sequence portions of the target DNA insert for quality control of library construction. As will be described below and a later sector of this disclosure, a variant of the *pSLGVT*-plasmid series, pSLGVT-3, utilizes these primer sites for high-throughput multiplex DNA sequencing of oligomerized GVT-pairs.

DNA Cloning Cassette 2 is produced from two complementary chemical synthesized oligonucleotides annealed to form a double strand structure with two terminal asymmetric 5' protruding cohesive ends, 5'-GAGT-3' and 5'-TCAC-3', for directed ligation of the cassette to the 5'-ACTC-3' overhangs of *Kan* module and to the 5'-GTGA-3' overhang of replicon module *(P15A-m* or *P15-e),* respectively. A pair of inverted *Eco*31 I sites on DNA cassette 2 produces a pair of 5'-TCAG-3' overhangs on the vector and provides alternate site to receive the GVT-adaptor ligated target DNA for the production of GVT-pairs. A *Sal* I site is situated between the set of *Eco*31 I sites for cloning a stuffer DNA fragment to help monitor the progress of *Eco*31 I digestion in preparation of the vector to receive target DNA. Flanking the *Eco*31 I sites are primer-binding sites for the M13 forward and M13 reverse sequencing primers. These primers sites are used to sequence portions of the target DNA insert for quality control of library construction. As will be described below and elsewhere in this disclosure, a variant of the *pSLGVT*-plasmid series, *pSLGVT-3*, utilizes these primer-binding sites for high-throughput multiplex DNA sequencing of oligomerized GVT-pairs.

Plasmid *pSLGVT-1* is constructed by the two-part ligation strategy. The *P15A-m* replicon module is incubated with DNA Cloning Cassette 1. In a separate ligation reactions, the Kan module is incubated with DNA Cloning Cassette 2. After one-hour incubation the two ligation reactions are combined to assemble the desired circular product. Plasmid *pSLGVT-2* is produced by a similar manner but with the *P15A-e* replicon module replacing the *P15A-m* replicon module in the initial ligation reaction.

An alternative route to the construction of *pSLGVT* series of plasmids is through chemical synthesis whereby the plasmids are assembled from a series of chemically synthesized oligonucleotides.

Plasmid *pSLGVT-3* of the present invention represents a novel approach for efficient construction of a family of multiplex DNA sequencing vectors for sequencing oligomerized GVT-pairs and other DNA segments. Plasmid *pSLGVT-3* is constructed by replacing the *P15A* replicon module on the *Bpi I* - *Fag* I fragment of *pSLGVT-2* with a fragment terminating with those sites containing the replicon derived from the pUC plasmid. The *pUC* replicon was derived from the low copy number *ColE1* replicon where a single base mutation in the *Ori* combined with the deletion of the rop regulator resulted in increased plasmid copy number from -20 copies to greater than a thousand copies per cell (Vieira and Messing, 1982). The high copy number of *pSLGVT-3* would facilitate template preparation for high-throughput DNA sequencing of oligomerized GVT-pairs. A salient feature *pSLGVT-3* is the aforementioned pairs of inverted type IIS restriction enzyme sites residing in DNA cassettes 1 and 2. Digestion of *pSLGVT-3* with *Esp*3 I and *Eco*31 I creates two DNA vector segments with asymmetric cohesive ends for the targeted and directed ligation of two independent sets of oligomerized GVT-pair segments allowing four separate sequencing reads from each of the four primer-binding sites present in DNA cassettes 1 and 2. Conventional sequencing vectors typically carry one insert and can support only two sequencing reads.

### 6. GVT-PAIR PRODUCTION

As used herein, fosmid, BAC and other episomal elements are referred collectively as plasmids, the method described below for GVT-pair generation is based the previously described illustrative examples of the GVT-DNA cassettes and GVT-adaptors. In certain embodiments, target DNA for GVT-pair production are fragmented randomly by mechanical or enzymatic means to produce fragments of a desired size for GVT-pair production. In other embodiments, target DNA are digested to completion with one or more restriction endonucleases in separate reactions or in combination to cleave target DNA at specified sites to produce a population of DNA fragments for production of GVT-pairs as described in this disclosure. For target DNA digested with enzymes that create cohesive ends, the dephosphorylated insert DNA may be cloned directly into a site between a pair of type IIS or type IIG sites of a suitably modified vector without the need of an adaptor. In yet another embodiment, target DNA are digested to completion with one or more restriction endonucleases and are fractionated to a desired size for use in GVT-pair production.

Target DNA for GVT-production having "ragged" ends are repaired using T₄ DNA polymerase and are dephosphorylated to prevent self-ligation of inserts during ligation of insert to the GVT-adaptor. Likewise, target DNA bearing cohesive ends are dephosphorylated before ligation to a suitable GVT-adopter bearing complementary ends. GVT-adaptor ligated DNA are passed through an appropriate ChromaSpin™ column (Clontech, Mountain View, CA) to remove unligated adaptor before ligation of adaptor-ligated target DNA to a GVT production vector. In certain embodiments, target DNA are size-selected to a desired length by gel electrophoresis or by other means prior to ligation of inserts to GVT-adaptor and subsequent ligation into a GVT-production vector such as pSLGVT-1 and pSLGVT-2 described in the present invention.

Ligation conditions for optimizing inter-molecular ligation of a vector to an insert followed by intra-molecular ligation to yield a circular molecule have been described for DNA segments over a range of fragment lengths (Collins and Weissman, 1984; Dugaiczyk et al, 1975; Wang and Davidson, 1966). General methods for ligating nucleic acid molecules, transfection into host cell and for construction of plasmid-based libraries are known to those that are skilled in the art. See, for example, Birren et al (In: Bacterial Artificial Chromosomes in Genome Analysis: A Laboratory Manual, CSH Press, New York, 1999), Ausubel et al (eds) (In: Short Protocols in Molecular Biology, 3rd Ed, John Wiley & Sons, New York 1995), and Sambrook et al (In: Molecular Cloning: A Laboratory Manual 2nd Ed, CSH press, New York, 1989). Ligated DNA is introduced into host cells by electroporation or by transfection. The propagation of methylated target DNA such as genomic DNA or cDNA synthesized by certain protocols that make use of methylated nucleotide analogues requires host cell strains with inactive *mcr* and *mrr* alleles. Suitable host strains include: 10G (Lucigen, Middleton, WI); *XL1-Blue MR* and *XL2Blue MRF' (Stratagene,* La Jolla, CA). Electroporated or transfected cells are plated onto 10 cm diameter agar plates at a density of ∼20,000 colonies per plate under the appropriate drug selection to yield the primary library. An alternative method is to grow the transfected cells in liquid culture while exercising care not to overgrow cells to encourage clonal selection. The total number of clones under culture should reflect the number of GVT-pairs required by the study design. Cells are harvested and the plasmids isolated for the subsequent step described below.

As a general procedure, purified plasmids bearing target DNA insert are digested with either *Mme* I*, Cst*M I*, NmeA* III, or *Eco*P15 I (New England Biolabs, Ipswich, MA) to generate the GVT in accordance with the experimental design. The ends of the newly created GVTs are repaired with T₄ DNA polymerase to render the digested ends blunt. Linearized plasmids with the newly created GVTs attached are purified away from the excised remnant of the intervening inserts by gel electrophoresis and the purified products are circularized by blunt-end ligation to yield the primary GVT-pair library. An alternative method for recircularizing the plasmids that avoids the need to repair DNA ends makes use of an adaptor bearing all 16-fold two-base pair degenerate 3'-overhangs or 5'-overhangs produced by *Mme* I*, Cst*M I, *Nme*A III, or *Eco*P15 digestion, respectively. The method would increase the length GVT produced by *Mme* I digestion from 18-bp to 20-bp but would not increase the length of *Eco*P15 I produced GVTs since *Eco*P15 I digestion creates 2-bp 3'-recessed ends that are filled in during repair by T₄ DNA polymerase prior to plasmid recircularization to the generate the GVT-pair. The use of an adaptor to recircularize the plasmid would increase the overall unit length of the resulting GVT-pairs with extraneous sequences with a resulting negative impact on sequencing economy of the oligomerized GVT-pairs.

Circularized plasmids are introduced into host cells and plated at a density of ∼20,000 colonies per 10 cm plate or grown in liquid culture under selection to yield the primary GVT-pair library. Purified plasmids from the primary GVT-pair library are digestion with an enzyme that cleaves both sides of the GVT-pairs to excise the GVT-pair from the plasmid. In the illustrated examples of the GVT-adaptors used for library construction, *Bam*H I or *Pst* I are used to excise the GVT-pairs from the *Mme* I or EcoP15 I generated GVT-pair libraries, respectively. Using a similar adaptor design, the enzymes *Bsp*T I or *Kas* I can be used to excise GVT-pairs from *Cst*M I or *NmeA* III generated GVT-pair libraries, respectively. The general structure of an excised GVT-pair generated by either *Mme* I or *Eco*P15 I digestion followed by blunt-end ligation is shown below:

### EXAMPLE NO 7: Structure of a GVT-pair monomer generated by Mme I digestion, intramolecular ligation and excision by BamH I digestion.

"18N-18N" represents the two juxtaposed 18-bp GVTs of a GVT-pair created from target DNA digested with *Mme* I. The pair of *Mme* I recognition sites on the monomer is underlined. The remaining portions of the 52-bp monomer, including the underlined *Mme* I sites, comprise a common "framework". The 52-bp GVT-pair monomer is separated by electrophoresis on a 5% poly-polyacrylamide gel and is purified and oligomerized for sequencing.

### EXAMPLE NO 8: Structure of GVT-pair monomer generated EcoP15 I digestion, intramolecular ligation and excision by Pst I digestion.

"27N-27N" represents the two juxtaposed 27-bp GVTs of a GVT-pair created from target DNA digested with *Eco*P15 I. The pair of *Eco*P15 I recognition sites on the monomer is underlined. The remaining portions of the 70-bp monomer, including the underlined *Eco*P15 I sites, comprise a common "framework." The 70-bp GVT-pair monomer is separated by electrophoresis on a 5% poly-polyacrylamide gel and is purified and oligomerized for sequencing.

### 7. PRODUCTION OF OLIGOMERIZED GVT-PAIR MONOMERS FOR EFFICIENT DNA SEQUENCING

DNA sequence tags are typically oligomerized and cloned into a sequence vector as an extended oligomer for economic use DNA sequencing resources. The present invention provides efficient methods to create oligomers of DNA tags and assemble the oligomerized DNA segments into an improved sequencing vector. Typically, DNA sequence tag monomers are constructed with termini bearing symmetric cohesive ends such as *Bam*H I or *Pst* I in the examples shown. However, the commonly used procedures for producing and cloning oligomerized sequence tag monomers are inherently inefficient due the creation of unproductive circular products during the oligomerization reaction and during the ligation of the insert into vector. As described herein, a novel and preferred method to produce and to clone oligomerized sequence tags is outlined below. The improved procedure makes use of an "initiator adaptor", which can initiate oligomerization of monomers and allows cloning the oligomeric product into a vector, but at the same time prevents circularization of the oligomerized DNA. Four illustrative example of suitable initiator adaptors are shown below:

### EXAMPLE NO 9: Initiator Adaptor GACA-Bam for BamH I oligomers.

### EXAMPLE NO 10: Initiator Adaptor GACA-Pst for Pst I oligomers.

### EXAMPLE NO 11: Initiator Adaptor CTGA-Bam for BamH I oligomers.

### EXAMPLE NO 12: Initiator Adaptor CTGA-Pst for Pst I oligomers.

Initiator adaptors are produced from two complementary chemically synthesized oligonucleotides annealed to form the illustrated double strand adaptors. At one terminus, the adaptors have a palindromic cohesive complementary end for ligation to either *Bam*H I or *Pst* I generated sequence tag monomers and initiate oligo formation. An asymmetric cohesive end (either 5'-GACA-3' or 5'-CTGA-3') is present at the other adaptor terminus for specific ligation into one or the other cloning site on the multiplex sequencing vector, *pSLGVT-3.* The unique design of *pSLGVT-3* and other plasmids of *the pSLGVT* series have the capacity to carry two independent DNA inserts.

Complementary cohesive end to the monomer at only one terminus of the initiation adaptor restricts ligation of monomer and the growth of the oligomer in one direction, thereby minimizes the formation of unproductive circular molecules. The lower strand of the initiation adaptors is unphosphorylated to prevent adaptor dimer formation. Oligomer formation is carried out in the presence of excess GVT-pair monomers to initiator adaptor in a ligation reaction that is allowed to go to completion. The principal products produced are a collection of oligomerized monomers "capped" at both ends by initiator adaptor. The ratio of DNA monomer to initiator adaptor dictates the overall size range of the final oligomerized product. A productive ratio is derived by titration using as a starting point, one part initiation adaptor to N parts monomer; where N equals (the average number of monomer desired in the final product plus 2)/2. If necessary, several ligation reactions employing a range of initiator adaptor to monomer ratios can be pooled and desired length product purified by gel electrophoresis. Conditions are chosen to yield oligomerized species from the GAGC- and GTGA-initiator adaptors comprising approximately twenty five to thirty copies (∼1.6 to 2 kb in length), which are purified on 1.5% agarose gel and cloned into the two sites of sequencing vector, *pSLGVT-3.*

### 8. CLONING OLlGOMERIZED GVT-PAIR MONOMERS INTO MULTIPLEX SEQUENCING VECTOR, pSLGVT-3

As used herein, the term multiplex sequencing vector refer to a plasmid vector optimized for Sanger dideoxy sequencing (Sanger et al, 1977) that has the capacity to carry an independent insert in each of two DNA cloning cassettes resulting in four sequencing reads from each of four primer binding sites.

*pSLGVT-3* (or its low copy number variant, *pSLGVT*-2) is digested with *Eco*31 I and *Esp*3 I to produce to yield two vector segments, which are purified by gel electrophoresis for use. Vector segment 1 comprises the plasmid replicon module and has 5'TCAG-3' and a 5'-TGTC-3' cohesive ends. Vector segment 2 comprises the Kan module and has 5'-TGTC-3' and 5'-TCAG-3' overhangs. Vector segment 1 is ligated with equal molar equivalent of oligomerized GVT-pairs produced by initiator adaptor GACA-. In a separate reaction, vector segment 2 is ligated to equal molar equivalent of oligomerized GVT-pairs produce by initiator adaptor CTGA-. After one-hour incubation the two ligation reactions are combined and reincubated to assemble the desired circular product comprising two independently derived inserts of oligomerized GVT-pairs, ligated between the two vector segments.

A typical sequence read lengths of 600 to 800 bp is sufficient to determine the sequence of at least 10 GVT-pairs. Based on the determination of 10 GVT-pairs per sequencing read and four sequencing reads from a single template, a single plasmid template of the present invention would generate the sequences of more than 40 GVT-pairs. Fosmid paired-end mapping with end-pair spacing of 40 kb requires 75,000 fosmid end-pairs spaced hypothetically end to end to cover the human genome at a cost of 75,000 fosmid template preparations and 150,000 sequencing reads. In comparison, one-fold coverage of the Human Genome at similar 40 kb spacing between GVTs by the use of the present invention would require 75,000 GVT-pairs produced at a cost of only 7,500 sequencing reads, and 1,875 plasmid template preparation. For a similar level of genomic coverage and resolution, the methods of the present invention enjoy a factor of twenty or more reduction in sequencing reads and a factor of forty or more reduction in template preparation when compared to the fosmid paired-end method of Tuzun et al (2005).

### PREFERRED EMBODIMENTS OF INVENTION

Evidence mounts that genetic structural variations comprise millions of base-pairs of heterogeneity in Man and is a major component of our genetic diversity some of which are almost certain to negotiate our interaction with the environment and play a role in disease, disease susceptibility or progression. The present invention relates to systems, methods, compositions, vectors, vector components and kits to create pairs of linked genomic sequence tags for the rapid generation of high-resolution genetic maps to identify such genomics variations.

In a preferred embodiment, the present invention identifies fine-structural-variations within a target genome through the creation of a plurality of GVT-pairs of unique genomic positional identifiers of defined spatial distance and orientations. The GVT-pairs collectively represent the genomic profile of the subject, which when compared with a reference sequence or to similarly produce genomic profiles of other target genomes, denote the presence of fine-structural-differences between nucleic acid populations. Genomic fine-structural-variations detectable by the present invention includes: deletion and insertions, duplication, inversions, translocation and other chromosomal rearrangements. The present invention offers means to identify these genomic features at a user defined resolution level dictated by the experimental design.

Assuming uniform distribution of the four bases, an 18-bp or 27-bp GVT of the present invention should occur by chance on average once every 4^{18 and} 4²⁷ base pairs, respectively, and should represent unique sequence identifiers in the human and other complex genomes. Unambiguous assignment of GVT to the genome improves when separation distance between GVTs is considered. For example, a GVT-pair comprising two spatially linked 18-bp GVTs produced from a size-fractionated target DNA population is effectively a 36-bp sequence tag. Similarly, a linked pair of 27-bp GVT is functionally a 54-bp sequence tag. Despite the tag length, it might not be possible to assign a very small set of GVT or GVT-pairs to a unique genomic position, such as those residing completely within repetitive elements. Regions of the genome that are retractile to analysis by the present invention are expected to be small and can be modeled by computational methods known in the art.

The common framework sequence present on each GVT-pair monomer allows unambiguous extraction of GVT-pair sequences from the high-throughput sequence data. Discordance between GVT-pairs to one or more reference sequences is revealed by alignment using MEGABLAST (Zhang et al, 2000) or similar computer programs. Discordance of the GVT-pair separation distance or orientation with the reference over a threshold level signals the presence of a structural difference between target and reference DNA. The threshold level is set by the experimental design, two standard deviations over the mean GVT separation distance being a reasonable default value. Deletions in the target DNA may be defined by two or more GVT-pairs spanning greater than two standard variations from the mean separation distance when compared to the reference sequence. Accordingly, insertions in the target DNA may be defined as sites where two or more GVT-pairs spanning less than two standard variations from the mean separation when compared to the reference sequence. Inversions in target DNA are defined as sites where two or more GVT-pairs having inconsistent orientation of their GVTs. Discordant GVT-pairs are manually curated and assessed before proceeding to validation by PCR, Southern blot hybridization analysis or by insert isolation and sequencing.

Target genomic nucleic acids of the invention can be derived from any source including: genomic DNA of eukaryotic, prokaryotic organisms, microbes, plastids, and viruses. Target genomic nucleic acids of the present invention can also be derived from RNA genome of organisms such as the RNA viruses through a reverse-transcription process to convert RNA to DNA. The choice of target nucleic acids for investigation may be influenced by prior knowledge of association of a particular chromosome or chromosome region with certain disease conditions described in the scientific literature. The present invention can utilize target DNA from isolated chromosomes or chromosome regions. The present invention can be used in broad whole genome-wide scans of patient cohorts at a range of resolutions to suit the study design. Methods for the purification of chromosome, chromosome segments, genomic DNA and RNA are known in the art. Also known in the art are methods to amplify nucleic acids by PCR or by other means to produce target DNA for analysis by the present invention.

Methods to cleave target DNA and to fractionate target the DNA to a desired size for setting the spatial distance between GVTs of a GVT-pair are described in an earlier section of this disclosure. Hydrodynamic shearing or partial enzymatic digestion of DNA with frequent cutting enzymes can be used to produce a population of DNA fragments with a high degree of overlapping fragments for maximal coverage every region of the target DNA. Alternatively, target DNA can be digested to completion with several restriction endonucleases in separate cleavage reactions and then size-fractioned to desired size classes for GVT-pair production. GVT-pairs produced from size-selected target DNA prepared by completion digestion with a single restriction endonuclease are nonoverlapping and cover only a portion of the target DNA complexity. Size-selected DNA fragments from complete enzymatic digestions with other restriction endonucleases can be used to cover gaps. Cleavage of target DNA randomly or in combination with complete enzymatic digestion to cover a genome of a given complexity can be modeled computationally by workers that are skill in the art to derive study design to make the optimal use of resources. Enzymes such *Bam*H I, *Hind* III, *Pst* I, *Spe* I and *Xba* I are insensitive to CpG methylation and would cleave mammalian genomic DNA at every site to produce GVT-pairs that accurately represent pairs of adjacent recognition sites for those enzymes. Other suitable enzymes that are insensitive to the effect of CpG methylation, overlapping CpG methylation or other kinds of DNA modifications that may influence nucleic acid analysis by the present invention have been described in the literature (McClelland et al, 1994; Geier et al, 1979; Kan et al, 1979; Hattman et al, 1978; Buryanov et al, 1978; May et al, 1975) and by major vendors of restriction endonucleases (Fermentas, Hanover, MD; New England Biolabs, Ispwich, MA). In certain embodiments, the use of enzymes whose cleavage of target DNA is sensitive to DNA modifications may be used to demarcate sites of modifications in the target DNA. For example, the present invention can identify sites of DNA methylation, which are known to regulate gene expression. For such an application, target DNA is digested to completion with a methylation sensitive restriction enzyme and GVT-pairs produced from the digested DNA. Sites of methylation are identified by discordance of the resulting GVT-pairs when compared to adjacent restriction sites on the reference sequence.

Discordant GVT-pairs are first manually curated before proceeding to a series of hierarchical filters for validation. In cases where the discordant GVT-pairs are produced from size-selected DNA derived from complete restriction endonuclease digestion, Southern blot analysis of target DNA and reference DNA digested with the same restriction endonuclease could be used to validate differences in marker distance between target and reference DNA. The GVTs are of sufficient length for use as PCR primers to isolate the intervening genomic sequence for shotgun sequencing to determine the precise nature of the structural variation.

It is believed the study of structural variations will shed new light on complex diseases, such as obesity and diabetes, whose development is triggered by the interactions of genes, genetic elements and the environment. The choice of nucleic acids for analysis by the present invention may be influenced by prior knowledge of association of a particular chromosome or chromosome region with certain disease conditions described in the scientific literature. The present invention can target DNA from isolated chromosomes or chromosome regions or tissue samples at high resolution. Alternatively, the present invention can be used in broad whole genome-wide scans of patient cohorts at a range of resolutions to suit the study design. The current technique of fosmid paired-end sequencing requires over a million sequence reads to analyze each individual at a moderate level of resolution and coverage, thereby limiting its use as a platform to scan large populations for association studies to find biomarkers that are diagnostic or prognostic to disease outcome as well as potential drug targets for medical intervention. The present invention offers a solution to these limitations, and as such it has the potential to create new medical diagnostics and to aid drug discovery.

In another preferred embodiment, fine-structural-variations identified by the present invention are used to design oligonucleotide array assays, microarray assays PCR-based assays and other diagnostic assays in the art to detect differences between nucleic acid populations. Present microarrays and oligonucleotide arrays are efficient platforms for detection of nucleic acid copy number alterations and single or small nucleotide polymorphisms but are not suited to detect other genomic changes that may contribute or are causal to disease. The identified products of the present invention enable the design of oligonucleotide and microarray assays and other diagnostic assays in the art to screen translocation, insertion, deletion, and inversion junctions that demarcate fine-structural-variations identified by the present invention. These assays could then be used to screen general population and large patient cohorts to determine the role of fine-structural-variations in complex diseases such as obesity, diabetes and many cancers, whose development is triggered by the interactions of multiple genetic and environmental factors. Other uses for these assays include but are not limited to the diagnosis or the differentiation between closely related species, strains, race or biotypes of microorganism with utilities in the fields of medical diagnostic and industrial microbiology.

In another preferred embodiment, the present invention is used to create high-resolution genomic maps to aid genomic assembly from shotgun DNA sequencing. A comprehensive set of unique genetic markers of defined separation distance or of adjacent restriction endonuclease sites would greatly facilitate whole genome sequencing efforts by providing a scaffold for genome assembly. It is expected that a number of GVT-pairs produced by the present invention that are discordant to the present build of the Human Genome Assembly (Build 35, May 2004) may not actually represent fine-structural variation in the target DNA, but rather reflect errors or gaps in the current Human Genome Assembly. Further compound the problem is that the current Genome Assembly is derived from DNA of pooled multiple donors. Reference sequences derived from single individuals that are representative of the range of human diversity are needed to move the genomics field forward. The utility offered by the present invention provides the means to do so.

In another preferred embodiment, the present invention is used to create high-resolution genomic maps to facilitate phylogenetic studies and for determining the genetic and functional relationship between closely related organisms. An aspect of the invention especially suited for this application makes use of GVT-pairs produced from target DNA digested to completion with one or more restriction endonucleases alone or in useful combination for GVT-pair production without a DNA size-fractionation step. Essentially, GVT-pairs constitute a genomic profile comprising pairs of positional markers that demarcate adjacent restriction endonuclease sites along the length of the target DNA. The identity of the GVT-pairs and their relative abundance can be used to create high-resolution genomic profiles that can be used to identify, differentiate and quantify the genome of origin within a complex medical or environmental DNA isolate. The so produced GVT-pairs also has utility in the area of industrial microbiology for identifying genomic differences causal to desirable traits, such as favorable growth rate and the production of useful secondary metabolites and recombinant proteins in closely related strains, biotypes, or race or genetically modified organisms. As such, the present can be used as a tool to aid strain improvement in the industrial production of microbial derived products. High resolution genomic maps produced by the present invention also offer a low cost and effective means to survey the nucleic acids of closely related pathogens to identify regions of variations to target detailed sequence analysis to identify pathogenic determinants that could be used for diagnosis and as drug targets for medical intervention.

In another preferred embodiment, the present invention can be use for genetic dissection of phenotype diversity in farm animals and agricultural crops to facilitate marker-assisted breeding. Farm animals are of a particular interest for identifying the complex genetic elements that contribute to the control of growth, energy metabolism, development, body composition, reproduction and behavior, as well as other traits sought by classical breeding. For a review see Andersson (2001). Most agricultural traits of interest are multi-factorial and are often controlled by an unknown number of quantitative trait loci (QTL). Microsatellite maps for genomic scans have been developed for the major farm animals. Association studies using these markers and the candidate gene approach are the two major strategies used for the identification of QTLs. The cloning of QTLs is challenging since the relationship between genotype and phenotype is considerable more complex than for the monogenic traits. However, it is possible to determine the QTL indirectly by progeny testing where the segregation of the QTLs is deduced using data from genetic markers and phenotypic variations among the progeny. At present, the molecular basis for most QTLs is as yet unknown. QTL mapping in Drosophila suggests that QTLs are often associated with sequence variations in the noncoding regions (MacKay, 2001). As in Man, it is expected that fine-structural-variations in the genomes of farm animals and crop plants will likely play an important role in phenotypic expression and interaction of the genome with the environment. The present invention provides the means to tabulate the comprehensive range of genomic structural diversity in farm animals and crop plants at low cost. The tabulated information would then enable the creation of oligonucleotide microarrays and other diagnostic platforms for use in association and linkage studies to identify and characterize the actual QTLs leading to marker-assisted breeding.

As the major pollinator, bees play a critical role in agriculture and in many parts of the world. Apiculture is another area that stands to benefit from the present invention. The honeybee is an economically important species suited to use genetic technology in breed development. Bees have a short generation time and produce large number of progeny. Lines are also readily propagated by artificial insemination. Bee strains exhibit broad phenotypic variations in productivity, disease resistance and behavioral traits, many of which are under complex genetically control. Important behavioral traits under genetic control include: aggression as exemplified by many African strains, foraging habits, honey yield and the so termed "hygienic" behavior. The "hygienic" trait is regulated by at least seven as yet undefined genetic loci, which in sum result in the cleaning behavior by the hive members to rid dead or disease broods as a primary defense against fungal and mite infestation, two major economic bee pathogens. A primary goal is to develop reliable diagnostic molecular markers that could be used in marker-assisted breeding to identify the desired progeny strains quickly and efficiently without the need for complicated and time consuming breeding experiments and field assays. Genetic maps and a reference sequence of the 200 mega-base size genome of *Apis mellifera* strain DH4 (Weinstock, 2006) is available for use by the present invention to provides efficient and low cost methods to survey genomes of multiple bee strains for fine-structural-variations at high resolution to correlate desired phenotype to genotype. The ability to survey multiple strains cost effectively is a key advantage offered by the present invention. For example, five-fold coverage of a 200 mega-base bee genome at a 10 kb resolution window would require only 10,000 sequence runs and 2,500 sequencing template preparations. The cost estimate is based on the sequence determination of 10 oligomerized GVT-pairs per sequencing runs and each vector template supporting four independent sequencing reactions.

In another preferred embodiment, the present invention can be used to identify genetic causes underlying neurological disorders and traits. It is generally believed that at least a component of many neurological disorders such as autism, bipolar disorder and schizophrenia have a complex non-Mendelian genetic component (Craddock and Jones, 2001; Owen and Craddock, 1996; Holzman and Matthysse, 1990). Complementing linkage and association studies in current use to identify the genomic components, the present invention provides means to assess the contributory role of genomic fine-structural-variations in neurological disorders and may lead to new methods for diagnosis, prognosis and patient management.

In another preferred embodiment, the present invention can be used to identify genetic causes underlying cancer thereby create means for diagnosis, prognosis, and therapeutic intervention. Virtually all cancers are due to abnormalities in DNA sequence, either inherited or acquired through somatic mutations during life. The prevailing tenet of oncogenesis is that together with environmental factors, accumulating DNA mutation alters gene expression or gene functions pass a critical functional threshold that allows clonal expansion, cellular invasion of surrounding tissues and the initiation of metastasis. One in three people in the Western World will develop cancer and one in five will die, making cancer the most common of the genetic diseases. The field historically began with the identification of potent onco- or tumor suppressor genes where a simple loss or gain of function due to small number of nucleotide changes to a locus was the major contributory factor to cancer. The field has since expanded to gene dosage where duplication or deletion of DNA segments resulting in alternation of gene copy number is the presumed cause of oncogenesis. The use of array CGH has been particularly useful for the detection of alteration in DNA copy number and the loss of heterozygosity in cancer cell lines and primary tumors. A comprehensive review of copy number analysis in cancer and a catalogue of somatic mutation in cancer and references therein can be found in under "The Cancer Genome Project" of the Sanger Institute (www.sanger.ac.uk/genetics/CGP/).

Most recently, the important role of genomic fine-structural-variations in oncogenesis is recognized. During the course of oncogenesis, the tumor genome accumulates a large number of rearrangements, including amplifications, deletion, translocations, inversions and the like, many of which contribute directly to tumor progression (Gray and Collins, 2000). Volik et al (2006) made use of a functional version of fosmid paired-end mapping to detect all changes in genomic architecture of a progressing tumor, in particular translocations and inversion events that are not detectable by array CGH. Their approach to dissect the breast cancer genome was most informative but was acknowledged by the investigators to be limited by the expense and resources required to obtain end-terminal sequences of the large number of BAC clones for each sample. The present invention offers low cost, high-resolution methods to overcome these deficiencies and to identify genomic fine-structural-variations not amendable to detection by array CGH. The present invention has sufficient low cost to enable use in broad surveys of cancer patient cohorts and for use to track the accumulation of genomic changes in tumor progression in individual patients. The ability to track genomic changes during tumor progression would have profound predictive value in clinical outcome, providing significant improvements in patient management.

In yet another preferred embodiment, the methods described herein can be used to identify mRNA processing variants. The concept of one gene encoding one protein is being superseded with one gene encoding a plurality of proteins, some of which have distinct functions that are medically relevant. The process appears highly regulated and is mediated in part through alternative processing of mRNA as well as by the differential usage of promoters, transcription terminators and post-translational processing. The process of trans-splicing, where two distinct mRNA transcripts recombine, further adds to the transcriptome complexity. The choice of target mRNA for use may be influenced by prior knowledge of certain disease condition, cell types, organ or developmental stage where certain mRNA variants may be of importance.

Those that are skilled in the art are familiar with method for mRNA isolation and the conversion of mRNA to cDNA. Within one aspect of the present invention, isolated RNA is converted to cDNA by reverse-transcription or reverse-transcription coupled with PCR by methods including the use of a random primer containing a restriction endonuclease such *Mme* I, *Cst*M I, *NmeA* III or *Eco*P15 I. The restriction site is situated on the primer such that digestion of the resulting double stand cDNA with the said endonuclease removes the primer sequence from the cDNA. Primer concentration is adjusted to yield average size products of 300 to 500 bp or in accordance to the experimental design. Following repair of the cDNA ends using T₄ DNA polymerase, the cDNA is dephosphorylated and ligated to a suitable GVT-adaptor and size-selected on a 5% poly-acrylamide gels for the production of GVT-pairs. mRNA processing variants are identified discordance of the GVT-pair with the NCBI Reference Sequence (RefSeq) or other databases. Processing variants are validated by PCR using primers derived from the discordant GVT-pairs.

### REFERENCES

Albertson DG and Pinkel D, 2003. Genomic microarrays in human genetic disease and cancer. Hum Mol Gen 12 Spec No 2: R145-R152.
Albertson DG et al, 2000. Quantitative mapping of amplicon structure by array CGH identifies CYP24 as a candidate oncogene. Nat Genet 25: 144-146.
Andersson L, 2001. Genetic dissection of phenotypic diversity in farm animals. Nat Rev 2: 130-138.
Bailey AB et al, 2002. Recent segmental duplications in the human genome. Science 297: 1003-1007.
Bignell GR et al, 2004. High-resolution analysis of DNA copy number using oligonucleotide microarrays. Genome Res 14: 287-295.
Bolivar F et al, 1977. Construction and characterization of new cloning vehicles. II multipurpose system. Gene 2: 95-113.
Boyd AC et al, 1986. Isolation and computer-aided characterization of Mme I, a type II restriction endonuclease from Methylophilus methylotrophus. Nuc Acids Res 13: 5255-5274.
Brennan C et al, 2004. High-resolution global profiling of genomic alterations with long oligonucleotide microarray. Cancer Res 64: 4744-4748.
Bujnicki JM, 2001. Understanding the evolution of restriction-modification systems: Clues from sequence and structure comparisons. Acta Biochimica Polonica 48: 935-967.
Buryanov YI et al, 1978. Site specific and chromatographics properties of E coli K12 and Eco RII DNA-cytosine methylases. FEBS Lett 88: 251-254.
Chang ACY and Cohen SN, 1978. Construction and characterization of amplifiable multicopy DNA cloning vehicles derived from the P15A cryptic miniplasmid. J Bacteriology 134: 1141-1156.
Check E, 2005. Patchwork people. Nature 437: 1084-1096.
Cheng Z et al, 2005. A genome-wide comparison of recent chimpanzee and human segmental duplications. Nature 437: 88-93.
Collins FS et al, 1987. Construction of a general human chromosome-jumping library, with application in cystic fibrosis. Science 235: 1046-1049.
Collins FS and Weissman SM, 1984. Directional cloning of DNA fragments at a large distance from an initial probe: A circularization method. Proc Natl Acad Sci (USA) 81: 6812-6816.
Craddock N and Jones I, 2001. Molecular genetics of bipolar disorder. Br J Psychiatry Suppl 41: S128-S133.
Deininger PL, 1983. Random subcloning of sonicated DNA: Application to shotgun DNA sequence analysis. Analyt Biochem 129: 216-223.
Dugaiczyk A et al, 1975. Ligation of Eco RI endonuclease-generated DNA fragments into linear and circular structures. J Mol Biol 96: 171-178.
Dunn JL et al, 2002. Genomic signature tags (GSTs): A system for profiling genomics DNA. Genome Res 12: 1756-1765.H
Feng T et al, 2002. Increased efficiency of cloning large DNA fragments using a lower copy number plasmid. BioTechniques 32: 992-998.
Feuk L et al, 2006. Structural variation in the human genome. Nature Rev 7: 85-97.
Fitzgerald MC et al, 1992. Rapid shotgun cloning utilizing the two base recognition endonuclease CviJ I. Nuc Acid Res 20: 3753-3762.
Geier GE and Modrich P, 1979. Recognition sequence of the dam methylase of Escherichia coli K12 and mode of cleavage of Dpn I endonuclease. J Biol Chem 254: 1408-1413.
Gonzalez E et al, 2005. The influence of CCL3L1 gene-containing segmental duplications on HIV-1/AIDS susceptibility. Science 307: 1434-1440.
Gray JW and Collins C, 2000. Genome changes and gene expression in human solid tumors. Carcinogenesis 21: 443-452.
Grindley NDF and Joyce CM, 1980. Genetic and DNA sequence analysis of the kanamycin resistance transposon Tn903. Proc Natl Acad Sci (USA) 77: 7176-7180.
Hamelin C and Yelle J, 1990. Gel and buffer effects on the migration of DNA molecules in agarose. Appl Theor Electrophor 1: 225-231.
Hattman S et al, 1978. Sequence specificity of the P1 modification methylase (M.Eco P1) and the DNA methylase (M.Eco dam) controlled by the Escherichia coli dam gene. J Mol Biol 126: 367-380.
Hayashi K et al, 1986. Regulation of inter- and intermolecular ligation with T4 DNA ligase in the presence of polyethylene glycol. Nuc Acids Res 14: 7617-7630.
Heffron F et al, 1978. In vitro mutagenesis of a circular DNA molecule by using synthetic restriction sites. Proc Natl Acad Sci (USA) 74: 6012-6016.
Heiskanen MA et al, 2000. Detection of gene amplification by genomic hybridization to cDNA microarrays. Cancer Res 60: 799-802.
Holzman PS and Matthysse S, 1990. The genetics of schizophrenia: A review. Pyschol Sci 1: 179-286.
Huang J et al, 2004. Whole genome DNA copy number changes by high density oligonucleotides arrays. Hum Genomics 1: 287-299.
Inazawa J et al, 2004. Comparative genomic hybridization (CGH)-arrays pave the way for identification of novel cancer-related genes. Cancer Sci 95: 559-563.
Kan NC et al, 1979. The nucleotide sequence recognized by the Escherichia coli K12 restriction and modification enzymes. J Mol Biol 130: 191-209.
Kinzler KW et al, 1995. Method for serial analysis of gene expression. U.S. Patent 5,695,937 (Issued Dec 9, 1997).
Kozdroj J and van Elsas JD, 2001. Structural diversity of microorganisms in chemically perturbed soil assessed by molecular and cytochemical approaches. J Microl Meth 43: 187-212.
Lok S, 2001. Methods for generating a continuous nucleotide sequence from non-contiguous nucleotide sequences. U.S. Patent 6,730,500 (Issued May 4, 2004).
Lucito R et al, 2003. Representational oligonucleotide microarray analysis: A high-resolution method to detect genome copy number variation. Genome Res 13: 2291-2305.
Mackay TFC, 2001. Quantitative trait loci in Drosophila. Nat Rev Genet 2: 11-20.
Matsumura H et al, 2003. Gene expression analysis of plant host-pathogen interactions by SuperSAGE. Proc Natl Acad Sci (USA) 100: 15718-15723.
May MA and Hattman S, 1975. Analysis of bacteriophage deoxyribonucleic acid sequences methylated by host- and R-factor-controlled enzymes. J Bacteriology 123: 768-770.
McClelland M et al, 1994. Effect of site-specific modification on restriction endonucleases and DNA modification methyltransferases. Nuc Acids Res 22: 3640-3659.
Mead, DA and Godiska R, 2001. Cloning vectors and vector components. U.S. Patent 6,709,861 (Issued Mar 23, 2004).
Melgar E and Goldthwait DA, 1968. Deoxyribonucleic acid nucleases: II. The effect of metals on the mechanism of action of deoxyribonuclease I. J Biol Chem 243: 4409-4416.
Mucke M et al, 2001. DNA cleavage by type III restriction-modification enzyme EcoP15I is independent of spacer distance between two head to head orientated recognition sites. J Mol Biol 312: 687-698.
Ng P et al, 2005. Gene identification signature (GIS) analysis for transcriptome characterization and genome annotation. Nat Meth 2: 105-111.
Owen MJ and Craddock N, 1996. Modern molecular genetic approaches to complex traits: Implications for psychiatric disorders. Mol Psychiatry 1: 21-26.
Peakman LJ et al, 2003. S-adenosyl methionine prevents promiscuous DNA cleavage by EcoP1 I type III restriction enzyme. J Mol Biol 333: 321-335.
Pheiffer BH and Zimmerman SB, 1983. Polymer-stimulated ligation: Enhanced blunt- or cohesive-end ligation of DNA or deoxyribooligonucleotides by T4 DNA ligase in polymer solutions. Nuc Acids Res 11: 7853-7871.
Pinkel D and Albertson DG, 2005. Array comparative genomic hybridization and its application in cancer. Nat Genet Suppl 37: S11-S17.
Pinkel D et al, 1998. High resolution analysis of DNA copy number variation using comparative genomic hybridization to microarrays. Nat Genet 20: 207-211.
Pinkel D et al, 1997. Comparative genomic hybridization. U.S. Patent No. 6,159,685 (Issued Dec 12, 2000).
Pinkel D et al, 1994. Comparative fluorescence hybridization to nucleic acid arrays. U.S. Patent No. 5,830,645 (Issued Nov 3, 1998).
Pollack JR et al, 2002. Microarray analysis reveals a major direct role of DNA copy number alternation in the transcriptional program of human breast tumors. Proc Natl Acad Sci (USA) 99: 12963-12968.
Pollack JR et al, 1999. Genome-wide analysis of DNA copy-number changes using cDNA microarrays. Nat Genet 23: 41-46.
Raghavendra N and Rao DN, 2004. Unidirectional translocation from recognition site and a necessary interaction with DNA end for cleavage by type III restriction enzyme. Nuc Acids Res 32: 5703-5711.
Redon R et al, 2006. Global variation in copy number in the human genome. Nature 444: 444-454.
Rouillard, J-M et al, 2001. Virtual genome scan: A tool for restriction landmark-based scanning of the human genome. Genome Res 11: 1453-1459.
Saha S et al, 2002. Using the transcriptome to annotate the genome. Nat Biotech 20: 508-512.
Sanger F et al, 1977. DNA sequencing with chain terminating inhibitors. Proc Natl Acad Sci (USA) 74: 5463-5467.
Schloter M et al, 2000. Ecology and evolution of bacterial microdiversity. FEMS Micobiol Rev 21: 647-660.
Schriefer LA et al, 1990. Low pressure DNA shearing: A method for random DNA sequence analysis. Nuc Acids Res 18: 7455.
Sistla S and Rao DN, 2004. S-adenosyl-L-methionine-dependent restriction enzymes. Crit Rev Biochem Mol Biol 39:1-19.
Snijders AM et al, 2001. Assembly of microarrays for genome-wide measurement of DNA copy numbers. Nat Genet 29: 263-264.
Szybalski W, 1997. Conditionally amplifiable BAC vector. U.S. Patent 5,874,259. (Issued Feb 23, 1999).
Szybalski E et al, 1991. Class-IIS restriction enzymes-A review. Gene 100: 13-26.
Tao Q and Zhang, H-B, 1998. Cloning and stable maintenance of DNA fragments over 300 kb in Escherichia coli with conventional plasmid-based vectors. Nuc Acids Res 21: 4901-4909.
Tuzun E et al, 2005. Fine-scale structural variation of the human genome. Nat Genet 37: 727-732.
Velculescu VE et al, 1995. Serial analysis of gene expression. Science 270: 484-487.
Vieira J and Messing J, 1982. The pUC plasmids, an M13mp7-derived system for insertion mutagenesis and sequencing with synthetic universal primers. Gene 19: 259-268.
Volik S et al, 2006. Decoding the fine-scale structure of a breast cancer genome and transcriptome. Genome Res 16: 394-404.
Wang JC and Davidson N, 1966. On the probability of ring closure of lambda DNA. J Mol Biol 19: 469-482.
Weinstock GM et al, 2006. Insights into social insects from the genome of the honeybee Apis mellifera. Nature 443: 931-949.
Wimmer K et al, 2002. Combined restriction landmark genomic scanning and virtual genome scans identify a novel human homeobox gene, ALX3, that is hypermethylated in neuroblastoma. Genes Chromosomes & Cancer 33: 285-294.
Zhang Z et al, 2000. A greedy algorithm for aligning DNA sequencing. J Computational Biol 7: 203-214.
Zimmerman SB and Pheiffer BH, 1983. Macromolecular crowding allows blunt-end ligation by DNA ligases from rat liver or Escherichia coli. Proc Natl Acad Sci (USA) 80: 5852-5856.

## Claims

1. A method for nucleic acid mapping and identification of fine structural variations in nucleic acids and utilities, wherein two genomic variation tags (GVTs) are juxtaposed and the two constituent members of the tag pair (GVT-pair) are unique positional markers of a defined separation distance in a target nucleic acid molecule, the method comprising:
- fragmenting DNA of a target population either randomly or at defined sites and then purifying the fragmented DNA sample to a predetermined size;
- ligating a DNA adaptor having one or more restriction endonuclease recognition sites to both ends of a fragmented target DNA insert;
- cloning the target DNA linked adaptor to a circular modular vector;
- digesting the adaptor using a restriction endonuclease, which recognizes a restriction endonuclease binding site within the adaptor sequence, to cleave the target DNA insert at a defined distance from each end of the target DNA insert to create two sequence tags (GVTs) comprising terminal sequences of the target DNA insert that are attached to the linearized modular vector backbone; and
- recircularizing the linearized modular vector backbone with the attached GVTs to obtain a circular molecule bearing a GVT pair comprising two juxtaposed GVTs;
- excising the newly created GVT pair from the vector;
- producing oligomerized GVT pair monomers for efficient DNA sequencing.

2. A method for nucleic acid mapping and identification of fine structural variations in nucleic acids and utilities, wherein two genomic variation tags (GVTs) are juxtaposed and the two constituent members of a tag pair are unique positional markers flanking two adjacent and cleavable restriction endonuclease sites, for one or more given restriction endonucleases, along the length of a population of target nucleic acid molecules, the method comprising:
- fragmenting DNA of a target population either randomly or at defined sites and then purifying the fragmented DNA sample to a predetermined size;
- cloning the digested target DNA inserts into a circular modular vector at a location flanked by a pair of sites for a type IIS, type IIG, or type III restriction endonuclease;
- cleaving the insert DNA at a defined distance from each end of the target DNA inserts, thereby creating two sequence tags (GVTs), comprising terminal sequences of the target DNA inserts that are attached to the linearized vector backbones; and
- recircularizing the vector backbones with the attached GVTs to form a circular molecule each bearing a GVT-pair comprising two juxtaposed GVTs;
- excising the newly created GVT pair from the vector;
- producing oligomerized GVT pair monomers for efficient DNA sequencing.

3. The method of claim 1 or 2, wherein the oligomerized GVT pair monomer is created through the controlled and ordered ligation of short DNA monomers possessing palindromic, rotationally equivalent cohesive ends to yield an oligomeric product bound at both ends by an initiation adaptor, comprising the steps of:
- forming an oligomer of DNA monomers initiated from an initiation adaptor wherein one adaptor terminus has a nonpalindromic cohesive end that cannot self-ligate, but can adhere to a vector, while the other adaptor terminus has a cohesive end that is unphosphorylated to prevent adaptor dimer formation and is complementary to the cohesive ends of the DNA monomer for ligation to monomer to initiate oligomer formation; and
- terminating oligomer growth upon ligation of either a free initiation adaptor to the oligomer formed by adding the DNA monomers or by ligation of another oligomer initiated by the initiation adaptor;
- wherein the oligomer so formed has an average length regulated by a molar ratio of DNA monomer to initiation adaptor measured when commencing formation of the oligomer.

4. The method of claim 1 or 2, wherein the circular modular vector are created through the following steps:
- providing two modular vector segments or modules of which the first module comprises a drug selectable marker and the second module comprises a replicon for plasmid replication and a terminal portion of each module having a type IIS endonuclease cleavage site that produces unique nonpalindromic cohesive end for the excision and targeted replacement of vector modules to create new vector functionalities;
- joining one end of the first and second modules in a DNA cassette comprising a recognition site which upon digestion with the endonuclease creates a pair of nonpalindromic cohesive ends on the vector for ligation to a DNA insert; and
- connecting the other end of the first and second modules to yield a circular molecule in a second DNA cassette comprising a second cloning site comprising another pair of restriction endonuclease recognition sites, which upon digestion with the endonuclease creates a pair of nonpalindromic cohesive ends on the vector that is distinct from those of the first cloning site, for ligation to a second and distinct recipient insert, and the cloning site is flanked on both sides by different DNA sequencing primer binding sites to prime Sanger dideoxy sequencing reactions into the recipient DNA insert; wherein the vector is free of recognition sites for *Mme* I*, Cst*M I, *Nme*A III, *Eco*P15 I*, Pst* II, *Bam*HI*, Pst*I*, Bsp*T I, or *Kas* I and the vector insert cloning sites comprise *Eco*31 I and *Esp*3 I recognition sites.

5. A method according to claim 1 or 2, wherein the two constituent member of the tag pair flank two adjacent and cleavable restriction endonuclease sites for one or more restriction endonucleases in the target nucleic acid molecule.

6. The method of claim 1 or 2, wherein the target DNA insert is selected from a group consisting of genomic DNA, cDNA, viral DNA, microbial DNA, plastid DNA, chemically synthesized DNA, DNA product of nucleic acid amplification, and DNA transcribed from RNA.

7. The method of claim 1, wherein the target DNA is fragmented randomly by the application of mechanical force or partial digestion with one or more enzymes.

8. The method of claim 1 or 2, wherein the target DNA is fragmented by complete digestion of using one or more restriction endonucleases alone or in combination.

9. The method of claim 1 or 2, wherein the fragmented target DNA is size fractionated.

10. The method of claim 1 or 2, wherein the fragmented target DNA is not size fractionated.

11. The method of claim 1 or 2, wherein the restriction endonuclease to create the GVT is a type IIS or type IIG restriction endonuclease selected from the group comprising of *Mme* I, *Nme*A III, *Cst*M I, *Bce*A I, *Bpm* I*, Bpu*E I, *Bsg* I, *Bsm*F I, *Bst*V1 I, *Eco*57 I, *Eco*57M I, and *Gsu* I.

12. The method of claim 1 or 2, wherein the type IIS or type IIG restriction endonuclease is *Mme* I.

13. The method of claim 1 or 2, wherein the restriction endonuclease to create the GVT is a type III restriction endonuclease selected from a group comprising of *Eco*P15 I, *Eco*P1 I, *Pst* II, *Hind* fIII, *Sty*LT I, *LIa*F I, *Bce*S I, *Hine* I, *Pha*B I, Hpy790545P, *Hpy*790639 I, and *Hpy*AXIP.

14. The method of claim 1 or 2, wherein the type III restriction endonuclease is *Eco*P15 I.

15. The method of claim 1 or 2, wherein the type IIS or type IIG restriction endonuclease to create the GVT recognize a six or more base pair uninterrupted recognition sequence.

16. The method of claim 1 or 2, wherein the type III restriction endonuclease to create the GVT recognize a six or more base pair of uninterrupted recognition sequence.

17. The method according to claim 4, wherein the selection marker is free of *Mme* I, *Cst*M I, *NmeA* III, *Eco*P15 I, *Pst* II, *Bam*H I, *Pst* I, *BspT* I, or *Kas* I restriction endonuclease sites.

18. The method according to claim 4, wherein the selection marker is the *Kan* gene.

19. The method according to claim 4, wherein the selection marker is the *Amp* gene.

20. The method according to claim 4, wherein the plasmid replicon is free of *Mme* I, *Cst*M I, *NmeA* III, *Eco*P15 I, *Pst* II, B*am*H I, *Pst* I, *Bsp*T I, or *Kas* I restriction endonuclease sites.

21. The method according to claim 4, wherein the plasmid insert cloning sites are created by digestion with type II, type IIS or type IIG restriction endonuclease that recognizes a six or more base pair uninterrupted sequence.

## Patentansprüche

1. Verfahren zum Kartieren von Nukleinsäuren und zum Identifizieren von feinstrukturellen Variationen in Nukleinsäuren und Nukleinsäuren untersuchenden Anwendungen, wobei zwei genomische Variations-Tags (GVTs) nebeneinander liegen und die beiden konstitutiven Elemente des Tag-Paares (GVT-Paar) spezifische Positionsmarker für einen festgelegten Trennungsabstand in einem Nukleinsäure-Zielmolekül sind, wobei das Verfahren umfasst:
- Fragmentieren der DNA einer Zielpopulation, entweder zufällig oder an festgelegten Stellen, und anschließendes Reinigen der fragmentierten DNA-Probe auf eine vorbestimmte Größe;
- Ligieren eines DNA-Adapters, der eine oder mehrere Erkennungsstellen für Restriktionsendonukleasen an beiden Enden von einem fragmentierten Insert der Ziel-DNA aufweist;
- Klonieren des mit der Ziel-DNA verbundenen Adapters in einen ringförmigen, modular aufgebauten Vektor;
- Verdauen des Adapters unter Verwendung einer Restriktionsendonuklease, die eine Restriktionsendonuklease-Bindungsstelle innerhalb der Adaptersequenz erkennt, zum Spalten des Ziel-DNA-Inserts in einem festgelegten Abstand zu jedem Ende von dem Ziel-DNA-Insert, um zwei Sequenz-Tags (GVTs) zu erzeugen, welche die endständigen Sequenzen von dem Ziel-DNA-Insert umfassen, die an das linearisierte, modular aufgebaute Vektorrückgrat angehängt sind; und
- Rezirkularisieren des linearisierten, modular aufgebauten Vektorrückgrates mit den angehängten GVTs zum Erhalten eines ringförmigen Moleküls, das ein GVT-Paar enthält, welches zwei nebeneinanderliegende GVTs umfasst;
- Herausschneiden des neu erzeugten GVT-Paares aus dem Vektor;
- Herstellen von oligomerisierten GVT-Paar-Monomeren zur leistungsfähigen DNA-Sequenzierung.

2. Verfahren zum Kartieren von Nukleinsäuren und zum Identifizieren von feinstrukturellen Variationen in Nukleinsäuren und Nukleinsäuren untersuchenden Anwendungen, wobei zwei genomische Variations-Tags (GVTs) nebeneinander liegen und die beiden konstitutiven Elemente eines Tag-Paares spezifische Positionsmarker sind, die zwei benachbarte und spaltbare Restriktionsendonukleasestellen, für eine oder mehrere vorgegebene Restriktionsendonukleasen, in einer Population von Nukleinsäure-Zielmolekülen in Längsrichtung flankieren, wobei das Verfahren umfasst:
- Fragmentieren der DNA einer Zielpopulation, entweder zufällig oder an festgelegten Stellen, und anschließendes Reinigen der fragmentierten DNA-Probe auf eine vorbestimmte Größe;
- Klonieren der verdauten Ziel-DNA-Inserts in einen ringförmigen, modular aufgebauten Vektor an einer Position, die durch ein Paar Restriktionsendonukleasestellen für Typ IIS-, Typ IIG- oder Typ III-Restriktionsendonukleasen flankiert ist;
- Spalten der eingebauten DNA in einem festgelegten Abstand zu jedem Ende der Ziel-DNA-Inserts, wodurch zwei Sequenz-Tags (GVTs) erzeugt werden, welche die endständigen Sequenzen der Ziel-DNA-Inserts umfassen, die an die linearisierten Vektorrückgrate angehängt sind; und
- Rezirkularisieren der linearisierten Vektorrückgrate mit den daran angehängten GVTs zum Bilden eines ringförmigen Moleküls, wobei jedes ein GVT-Paar enthält, welches zwei nebeneinanderliegende GVTs umfasst;
- Herausschneiden des neu erzeugten GVT-Paares aus dem Vektor;
- Herstellen von oligomerisierten GVT-Paar-Monomeren zur leistungsfähigen DNA-Sequenzierung.

3. Verfahren nach Anspruch 1 oder 2, wobei das oligomerisierte GVT-Paar-Monomer erzeugt wird durch die kontrollierte und aufeinanderfolgende Ligation kurzer DNA-Monomere, die palindrome, rotationssymmetrische, kohäsive Enden besitzen, wodurch ein oligomeres Produkt erhalten wird, das an beiden Enden an einen Translationsinitiations-Adapter gebunden ist, umfassend die Schritte von:
- Bilden eines Oligomers aus DNA-Monomeren, eingeleitet durch einen Translationsinitiations-Adapter, wobei ein Terminus des Adapters ein nicht-palindromes kohäsives Ende aufweist, das nicht selbstligierend ist, sich aber an einen Vektor anlagern kann, während der andere Terminus des Adapters ein kohäsives Ende aufweist, das nicht phosphoryliert ist, um die Dimerbildung des Adapters zu verhindern, und komplementär ist zu den kohäsiven Enden des DNA-Monomers für die Ligation an das Monomer, um die Oligomerbildung einzuleiten; und
- Terminieren des Oligomerwachstums durch Ligation von entweder einem freien Translationsinitiations-Adapter an das Oligomer, das durch Addition der DNA-Monomere gebildet wurde, oder durch Ligation von einem weiteren Oligomer, eingeleitet durch den Translationsinitiations-Adapter;
- wobei das derart gebildete Oligomer eine durchschnittliche Länge aufweist, die durch das molare Verhältnis, gemessen am Anfang der Oligomerbildung, von DNA-Monomer zu Translationsinitiations-Adapter reguliert wird.

4. Verfahren nach Anspruch 1 oder 2, wobei der ringförmige, modular aufgebaute Vektor durch die folgenden Schritte erzeugt wird:
- Bereitstellen von zwei modular aufgebauten Vektorsegmenten oder Vektormodulen, von denen das erste Modul einen über Arzneimittel selektierbaren Marker und das zweite Modul ein Replikon zur Plasmid-Replikation umfasst und ein terminaler Anteil von jedem Modul eine Spaltungsstelle für Typ IIS-Endonukleasen enthält, die spezifische, nicht-palindrome kohäsive Enden für das Ausschneiden und den zielgerichteten Austausch der Vektormodule produziert, um neue Vektor-Funktionalitäten zu erzeugen:
- Verbinden von einem Ende des ersten und des zweiten Moduls in einer DNA-Kassette, die eine Erkennungsstelle umfasst, welche nach Verdau mit der Endonuklease ein Paar nicht-palindrome kohäsive Enden an dem Vektor zur Ligation an ein DNA-Insert erzeugt; und
- Zusammenfügen des anderen Endes von dem ersten und dem zweiten Modul, um ein ringförmiges Molekül in einer zweiten DNA-Kassette zu ergeben, umfassend eine zweite Klonierungsstelle, umfassend ein weiteres Paar von Erkennungsstellen für Restriktionsendonukleasen, welche nach Verdau mit der Endonuklease ein Paar nicht-palindrome kohäsive Enden an dem Vektor erzeugt, die unterschiedlich zu denen der ersten Klonierungsstelle sind, zur Ligation an ein zweites und unterschiedliches Empfänger-Insert, und die Klonierungsstelle ist an beiden Seiten durch unterschiedliche Primer-Bindungsstellen für die DNA-Sequenzierung flankiert, um die Dideoxy-Sequenzierungsreaktionen nach Sanger in dem Empfänger-DNA-Insert zu starten; wobei der Vektor frei ist von Erkennungsstellen für *Mme* I, *Cst*M I, *Nme*A III, *Eco*P15 I, *Pst* II, *Bam*H I, *Pst* I, *Bsp*T I oder *Kas* I und die Klonierungsstellen des Vektor-Inserts *Eco*31 I- und *Esp*3 1-Erkennungsstellen umfassen.

5. Verfahren nach Anspruch 1 oder 2, wobei die beiden konstitutiven Elemente von dem Tag-Paar zwei nebeneinanderliegende und spaltbare Restriktionsendonukleasestellen für eine oder mehrere Restriktionsnukleasen in dem Nukleinsäure-Zielmolekül flankieren.

6. Verfahren nach Anspruch 1 oder 2, wobei das Ziel-DNA-Insert ausgewählt ist aus der Gruppe, die aus genomischer DNA, cDNA, viraler DNA, mikrobieller DNA, Plastid-DNA, chemisch synthetisierter DNA, DNA-Produkten der Nukleinsäure-Amplifikation und DNA, die von RNA transkribiert wurde, besteht.

7. Verfahren nach Anspruch 1, wobei die Ziel-DNA durch die Anwendung von einer mechanischen Kraft oder durch eine teilweise Verdauung mit einem oder mehreren Enzymen willkürlich fragmentiert ist.

8. Verfahren nach Anspruch 1 oder 2, wobei die Ziel-DNA mittels vollständiger Verdauung unter Verwendung von einer oder mehrerer Restriktionsendonukleasen alleine oder in Kombination fragmentiert ist.

9. Verfahren nach Anspruch 1 oder 2, wobei die fragmentierte Ziel-DNA größenfraktioniert ist.

10. Verfahren nach Anspruch 1 oder 2, wobei die fragmentierte Ziel-DNA nicht größenfraktioniert ist.

11. Verfahren nach Anspruch 1 oder 2, wobei die Restriktionsendonuklease zum Erzeugen des GVT eine Typ IIS- oder Typ IIG-Restriktionsendonuklease ist, ausgewählt aus der Gruppe, die aus *Mme* I, *Nme*A III, *Cst*M I, *Bce*A I, *Bpm* I, *Bpu*E I, *Bsg* I, *Bsm*F I, *Bst*V1 I, *Eco*57 I, *Eco*57M I und *Gsu* I besteht.

12. Verfahren nach Anspruch 1 oder 2, wobei die Typ IIS-oder Typ IIG-Restriktionsendonuklease *Mme* I ist.

13. Verfahren nach Anspruch 1 oder 2, wobei die Restriktionsendonuklease zur Erzeugung des GVT eine Typ III-Restriktionsendonuklease ist, ausgewählt aus der Gruppe, die aus *Eco*P15 I, *Eco*P1 I, *Pst* II, *Hind* fIll, StyLT I, *LlaF* I, *BceS* I, *Hine* I, *Pha*B I, *Hpy*790545P, *Hpy*790639 I und *Hpy*AXIP besteht.

14. Verfahren nach Anspruch 1 oder 2, wobei die Typ III-Restriktionsendonuklease *Eco*P15 I ist.

15. Verfahren nach Anspruch 1 oder 2, wobei die Typ IIS-oder Typ IIG-Restriktionsendonuklease zur Erzeugung des GVT eine aus sechs oder mehr Basenpaaren bestehende ununterbrochene Erkennungssequenz erkennt.

16. Verfahren nach Anspruch 1 oder 2, wobei die Typ III-Restriktionsendonuklease zur Erzeugung des GVT eine aus sechs oder mehr Basenpaaren bestehende ununterbrochene Erkennungssequenz erkennt.

17. Verfahren nach Anspruch 4, wobei der Selektionsmarker frei von Restriktionsendonukleasestellen für *Mme* I, *Cst*M I, *Nme*A III, *Eco*P15 I, *Pst* II, *Bam*H I, *Ps*t I, *Bsp*T I oder Kas I ist.

18. Verfahren nach Anspruch 4, wobei der Selektionsmarker das *Kan*-Gen ist.

19. Verfahren nach Anspruch 4, wobei der Selektionsmarker das *Amp*-Gen ist.

20. Verfahren nach Anspruch 4, wobei das Plasmid-Replikon frei von Restriktionsendonukleasestellen für *Mme* I, *Cst*M I, *Nme*A III, *Eco*P15 I, *Pst* II, *Bam*H I, *Pst* I, *BspT* I oder *Kas* I ist.

21. Verfahren nach Anspruch 4, wobei die Klonierungsstellen von dem Plasmid-Insert durch den Verdau mit Typ II-, Typ IIS- oder Typ IIG-Restriktionsendonukleasen erzeugt wird, die eine aus sechs oder mehr Basenpaaren bestehende ununterbrochene Sequenz erkennen.

## Revendications

1. Procédé de cartographie d'acides nucléiques et d'identification de variations structurales fines dans des acides nucléiques et applications, dans lequel deux marqueurs de variations génomiques (GVT) sont juxtaposés et les deux éléments constituants de la paire de marqueurs (paire de GVT) sont des marqueurs positionnels uniques d'une distance de séparation définie dans une molécule d'acide nucléique cible, le procédé comprenant :
- la fragmentation de l'ADN d'une population cible soit de manière aléatoire soit à des sites définis puis la purification de l'échantillon d'ADN fragmenté à une taille prédéterminée ;
- la ligature d'un adaptateur d'ADN ayant un ou plusieurs sites de reconnaissance d'endonucléase de restriction aux deux extrémités ou un insert d'ADN cible fragmenté ;
- le clonage de l'adaptateur lié d'ADN cible à un vecteur modulaire circulaire ;
- la digestion de l'adaptateur en utilisant une endonucléase de restriction, qui reconnaît un site de liaison d'endonucléase de restriction à l'intérieur de la séquence de l'adaptateur, pour cliver l'insert d'ADN cible à une distance définie de chaque extrémité de l'insert d'ADN cible pour créer deux marqueurs de séquence (GVT) comprenant les séquences terminales de l'insert d'ADN cible qui sont liées au squelette du vecteur modulaire linéarisé ; et
- la recircularisation du squelette de vecteur modulaire linéarisé avec les GVT liés pour obtenir une molécule circulaire portant une paire de GVT comprenant deux GVT juxtaposés ;
- l'excision de la paire de GVT nouvellement créée du vecteur ;
- la production de monomères de paires de GVT oligomérisés pour un séquençage efficace de l'ADN.

2. Procédé de cartographie d'acides nucléiques et d'identification de variations structurales fines dans des acides nucléiques et applications, dans lequel deux marqueurs de variations génomiques (GVT) sont juxtaposés et les deux éléments constituants d'une paire de marqueurs sont des marqueurs positionnels uniques flanquant deux sites d'endonucléase de restriction adjacents et clivables, pour une ou plusieurs endonucléases de restriction données, sur la longueur d'une population de molécules d'acides nucléiques cibles, le procédé comprenant :
- la fragmentation de l'ADN d'une population cible soit de manière aléatoire soit à des sites définis puis la purification de l'échantillon d'ADN fragmenté à une taille prédéterminée ;
- le clonage des inserts d'ADN cibles digérés dans un vecteur modulaire circulaire à un emplacement flanqué d'une paire de sites pour une endonucléase de restriction de type IIS, de type IIG, ou de type III ;
- le clivage de l'ADN inséré à une distance définie de chaque extrémité des inserts d'ADN cibles, créant ainsi deux marqueurs de séquence (GVT) comprenant les séquences terminales des inserts d'ADN cibles qui sont liées aux squelettes des vecteurs linéarisés ; et
- la recircularisation des squelettes de vecteurs avec les GVT liés pour former une molécule circulaire portant une paire de GVT comprenant deux GVT juxtaposés ;
- l'excision de la paire de GVT nouvellement créée du vecteur ;
- la production de monomères de paires de GVT oligomérisés pour un séquençage efficace de l'ADN.

3. Procédé selon la revendication 1 ou 2, dans lequel le monomère de paire de GVT oligomérisés est créé par la ligature contrôlée et ordonnée de monomères d'ADN courts possédant des extrémités cohésives palindromiques et équivalentes sur le plan rotationnel pour donner un produit oligomère lié aux deux extrémités par un adaptateur d'initiation, qui comprend les étapes consistant à :
- former un oligomère de monomères d'ADN initié par un adaptateur d'initiation, dans lequel une extrémité terminale de l'adaptateur a une extrémité cohésive non palindromique qui ne peut pas s'auto-ligaturer, mais qui peut adhérer à un vecteur, alors que l'autre extrémité terminale de l'adaptateur a une extrémité cohésive qui n'est pas phosphorylée pour empêcher la formation de dimères de l'adaptateur et est complémentaire des extrémités cohésives du monomère d'ADN pour la ligature au monomère pour l'initiation de la formation d'oligomères ; et
- la terminaison de la formation de l'oligomère par la ligature d'un adaptateur d'initiation libre à l'oligomère formé par ajout des monomères d'ADN ou par ligature d'un autre oligomère initié par l'adaptateur d'initiation ;
- l'oligomère ainsi formé ayant une longueur moyenne régulée par un rapport molaire du monomère d'ADN sur l'adaptateur d'initiation mesuré au début de la formation de l'oligomère.

4. Procédé selon la revendication 1 ou 2, dans lequel les vecteurs modulaires circulaires sont créés par les étapes suivantes :
- la fourniture de deux segments ou modules de vecteurs modulaires, le premier module comprenant un marqueur sélectif d'un médicament et le second module comprenant un réplicon pour la réplication de plasmides et une partie terminale de chaque module ayant un site de clivage d'endonucléase de type IIS qui produit une extrémité cohésive non palindromique unique pour l'excision et le remplacement ciblé de modules de vecteurs pour créer de nouvelles fonctionnalités de vecteurs ;
- la jonction d'une extrémité de chacun des premier et second modules dans une cassette d'ADN comprenant un site de reconnaissance qui, sous l'effet de la digestion avec l'endonucléase, crée une paire d'extrémités cohésives non palindromiques sur le vecteur pour la ligature à un insert d'ADN ; et
- la connexion de l'autre extrémité de chacun des premier et second modules pour donner une molécule circulaire dans une seconde cassette d'ADN comprenant un second site de clonage comprenant une autre paire de sites de reconnaissance d'endonucléase de restriction qui, sous l'effet de la digestion avec l'endonucléase, crée une paire d'extrémités cohésives non palindromiques sur le vecteur qui sont différentes de celles du premier site de clonage, pour la ligature à un second insert récepteur distinct, et le site de clonage est flanqué sur les deux côtés de différents sites de liaison d'amorce de séquençage d'ADN pour amorcer des réactions de séquençage didésoxy de Sanger dans l'insert d'ADN récepteur ; le vecteur étant dépourvu des sites de reconnaissance de *Mme* I, *Cst*M I, *Nme*A III, *Eco*P15 I, *Pst* II, *Bam*H I, *Pst* I, *BspT* I, ou *Kas* I et les sites de clonages d'inserts vectoriels comprenant les sites de reconnaissance *Eco*31 I et *Esp3* I.

5. Procédé selon la revendication 1 ou 2, dans lequel les deux éléments constituants de la paire de marqueurs flanquent deux sites d'endonucléase de restriction adjacents et clivables pour une ou plusieurs endonucléases de restriction dans la molécule d'acide nucléique cible.

6. Procédé selon la revendication 1 ou 2, dans lequel l'insert d'ADN cible est choisi dans un groupe constitué par l'ADN génomique, l'ADNc, l'ADN viral, l'ADN microbien, l'ADN plastidique, l'ADN chimiquement synthétisé, un produit d'ADN issu d'une amplification d'acide nucléique, et un ADN transcrit d'ARN.

7. Procédé selon la revendication 1, dans lequel l'ADN cible est fragmenté de manière aléatoire par l'application d'une force mécanique ou d'une digestion partielle avec une ou plusieurs enzymes.

8. Procédé selon la revendication 1 ou 2, dans lequel l'ADN cible est fragmenté par digestion complète en utilisant une ou plusieurs endonucléases de restriction seules ou en combinaison.

9. Procédé selon la revendication 1 ou 2, dans lequel l'ADN cible fragmenté est fractionné en classes de taille.

10. Procédé selon la revendication 1 ou 2, dans lequel l'ADN cible fragmenté n'est pas fractionné en classes de taille.

11. Procédé selon la revendication 1 ou 2, dans lequel l'endonucléase de restriction pour créer le GVT est une endonucléase de restriction de type IIS ou de type IIG choisie dans le groupe comprenant Mme I, NmeA III, *Cst*M I, *Bce*A I, *Bpm* I, *Bpu*E I, *Bsg* I, *Bsm*F I, *Bst*V1 I, *Eco*57 I, *Eco*57M I, et *Gsu* I.

12. Procédé selon la revendication 1 ou 2, dans lequel l'endonucléase de restriction de type IIs ou de type IIG est Mme I.

13. Procédé selon la revendication 1 ou 2, dans lequel l'endonucléase de restriction pour créer le GVT est une endonucléase de restriction de type III choisie dans un groupe comprenant *Eco*P15 I, *Eco*P1 I, *Pst* II, *Hind* fIII, *Sty*LT I, *LIa*F I, *Bce*S I, *Hine* I, *Pha*B I, *Hpy7*90545P, *Hpy*790639 I, et HpyAXIP.

14. Procédé selon la revendication 1 ou 2, dans lequel l'endonucléase de restriction de type III est *Eco*P15 I.

15. Procédé selon la revendication 1 ou 2, dans lequel l'endonucléase de restriction de type IIS ou de type IIG pour créer le GVT reconnaît une séquence de reconnaissance non interrompue de 6 paires de bases ou plus.

16. Procédé selon la revendication 1 ou 2, dans lequel l'endonucléase de restriction de type III pour créer le GVT reconnaît une séquence de reconnaissance non interrompue de 6 paires de bases ou plus.

17. Procédé selon la revendication 4, dans lequel le marqueur de sélection est dépourvu des sites d'endonucléase de restriction *Mme* I, *Cst*M I, *Nme*A III, *Eco*P15 I, *Pst* II, *Bam*H I, *Pst* h, *BspT* I, ou *Kas* I.

18. Procédé selon la revendication 4, dans lequel le marqueur de sélection est le gène *Kan.*

19. Procédé selon la revendication 4, dans lequel le marqueur de sélection est le gène Amp.

20. Procédé selon la revendication 4, dans lequel le réplicon de plasmide est dépourvu des sites d'endonucléase de restriction *Mme* I, *Cst*M I, *Nme*A III, *Eco*P15 I, *Pst* II, *Bam*H I, *Pst* I, *BspT* I, ou *Kas* I.

21. Procédé selon la revendication 4, dans lequel les sites de clonage de l'insert plasmidique sont créés par digestion avec une endonucléase de restriction de type II, de type IIS ou de type IIG qui reconnaît une séquence ininterrompue de 6 paires de bases ou plus.


### SEQUENCE LISTING

<110> LOK, Si
<120> METHODS FOR NUCLEIC ACID MAPPING AND IDENTIFICATION OF FINE-STRUCTURAL-VARIATIONS IN NUCLEIC ACIDS AND UTILITIES
<130> C 5 WO 3 EP
<150> PCT/CN2007/000001
   <151> 2007-01-04
<160> 28
<170> PatentIn version 3.3
<210> 1
   <211> 17
   <212> DNA
   <213> Artificial
<220>
   <223> artificial sequence
<400> 1
   gacacagagg atccaac 17
<210> 2
   <211> 13
   <212> DNA
   <213> Artificial
<220>
   <223> artificial sequence
<400> 2
   gttggatcct ctg 13
<210> 3
   <211> 17
   <212> DNA
   <213> Artificial
<220>
   <223> artificial sequence
<400> 3
   ctaggttgga tcctctg 17
<210> 4
   <211> 17
   <212> DNA
   <213> Artificial
<220>
   <223> artificial sequence
<400> 4
   gacacagact gcagcag 17
<210> 5
   <211> 13
   <212> DNA
   <213> Artificial
<220>
   <223> artificial sequence
<400> 5
   ctgctgcagt ctg 13
<210> 6
   <211> 17
   <212> DNA
   <213> Artificial
<220>
   <223> artificial sequence
<400> 6
   ctagctgctg cagtctg 17
<210> 7
   <211> 46
   <212> DNA
   <213> Artificial
<220>
   <223> artificial sequence
<400> 7
   aattggacaa gagacggaat attctagaac gatacgtctc ctgtcc 46
<210> 8
   <211> 46
   <212> DNA
   <213> Artificial
<220>
   <223> artificial sequence
<400> 8
   aattggacag gagacgtatc gttctagaat atgccgtctc ttgtcc 46
<210> 9
   <211> 56
   <212> DNA
   <213> Artificial
<220>
   <223> artificial sequence
<400> 9
   gagagacaag agacggcatc tcagtagtct agaagtgcac gatagcgtct cctgtc 56
<210> 10
   <211> 55
   <212> DNA
   <213> Artificial
<220>
   <223> artificial sequence
<400> 10
   aacgacagga gacgctatcg tgcacttcta gactactgag atgccgtctc ttgtc 55
<210> 11
   <211> 56
   <212> DNA
   <213> Artificial
<220>
   <223> artificial sequence
<400> 11
   gagtctgatg agaccctagc ctcttgagtc gaccactata catcaggtct cctcag 56
<210> 12
   <211> 56
   <212> DNA
   <213> Artificial
<220>
   <223> artificial sequence
<400> 12
   tcacctgagg agacctgatg tatagtggtc gactcaagag gctagggtct catcag 56
<210> 13
   <211> 20
   <212> DNA
   <213> Artificial
<220>
   <223> artificial sequence
<400> 13
   taatacgact cactataggg 20
<210> 14
   <211> 20
   <212> DNA
   <213> Artificial
<220>
   <223> artificial sequence
<400> 14
   attaaccctc actaaaggga 20
<210> 15
   <211> 19
   <212> DNA
   <213> Artificial
<220>
   <223> artificial sequence
<400> 15
   cacgacgttg taaaacgac 19
<210> 16
   <211> 20
   <212> DNA
   <213> Artificial
<220>
   <223> artificial sequence
<400> 16
   ggataacaat ttcacacagg 20
<210> 17
   <211> 48
   <212> DNA
   <213> Artificial
<220>
   <223> artificial sequence
<220>
   <221> misc_feature
   <222> (9)..(44)
   <223> n is a, c, g, or t
<400> 17
   gatccaacnn nnnnnnnnnn nnnnnnnnnn nnnnnnnnnn nnnngttg 48
<210> 18
   <211> 48
   <212> DNA
   <213> Artificial
<220>
   <223> artificial sequence
<220>
   <221> misc_feature
   <222> (9)..(44)
   <223> n is a, c, g, or t
<400> 18
   gatccaacnn nnnnnnnnnn nnnnnnnnnn nnnnnnnnnn nnnngttg 48
<210> 19
   <211> 66
   <212> DNA
   <213> Artificial
<220>
   <223> artificial sequence
<220>
   <221> misc_feature
   <222> (5)..(58)
   <223> n is a, c, g, or t
<400> 19
   gcagnnnnnn nnnnnnnnnn nnnnnnnnnn nnnnnnnnnn nnnnnnnnnn nnnnnnnnct 60
   gctgca 66
<210> 20
   <211> 66
   <212> DNA
   <213> Artificial
<220>
   <223> artificial sequence
<220>
   <221> misc_feature
   <222> (5)..(58)
   <223> n is a, c, g, or t
<400> 20
   gcagnnnnnn nnnnnnnnnn nnnnnnnnnn nnnnnnnnnn nnnnnnnnnn nnnnnnnnct 60
   gctgca 66
<210> 21
   <211> 18
   <212> DNA
   <213> Artificial
<220>
   <223> artificial sequence
<400> 21
   gacacacgtg ctagtccg 18
<210> 22
   <211> 18
   <212> DNA
   <213> Artificial
<220>
   <223> artificial sequence
<400> 22
   gatccggact agcacgtg 18
<210> 23
   <211> 22
   <212> DNA
   <213> Artificial
<220>
   <223> artificial sequence
<400> 23
   gacacacgtg ctagtccctg ca 22
<210> 24
   <211> 14
   <212> DNA
   <213> Artificial
<220>
   <223> artificial sequence
<400> 24
   gggactagca cgtg 14
<210> 25
   <211> 18
   <212> DNA
   <213> Artificial
<220>
   <223> artificial sequence
<400> 25
   ctgacacgtg ctagtccg 18
<210> 26
   <211> 18
   <212> DNA
   <213> Artificial
<220>
   <223> artificial sequence
<400> 26
   gatccggact agcacgtg 18
<210> 27
   <211> 22
   <212> DNA
   <213> Artificial
<220>
   <223> artificial sequence
<400> 27
   ctgacacgtg ctagtccctg ca 22
<210> 28
   <211> 14
   <212> DNA
   <213> Artificial
<220>
   <223> artificial sequence
<400> 28
   gggactagca cgtg 14